(19) Europäisches Patentamt
European Patent Office
Office européen des brevets

(11) Veröffentlichungsnummer: **0 263 521 B1**

(12) # EUROPÄISCHE PATENTSCHRIFT

(45) Veröffentlichungstag der Patentschrift: **11.12.91**

(51) Int. Cl.5: **C07K 7/20**, A61K 37/02

(21) Anmeldenummer: **87114702.1**

(22) Anmeldetag: **08.10.87**

(54) **Analoga von Gonadoliberin mit verbesserter Löslichkeit, Verfahren zu deren Herstellung, diese enthaltende Mittel und und ihre Verwendung.**

(30) Priorität: **09.10.86 DE 3634435**

(43) Veröffentlichungstag der Anmeldung:
**13.04.88 Patentblatt 88/15**

(45) Bekanntmachung des Hinweises auf die Patenterteilung:
**11.12.91 Patentblatt 91/50**

(84) Benannte Vertragsstaaten:
**AT BE CH DE ES FR GB GR IT LI LU NL SE**

(56) Entgegenhaltungen:
**FR-A- 2 465 486**

**CARBOHYDRATE RESEARCH, Band 89, Nr. 2, März 1981, Seiten 229-236, Elsevier Scientific Publishing Co., Amsterdam, NL; S. LAVIELLE et al.: "Synthesis of a glyotripeptide and a glycosomatostatin containing the 3-O-(2-acetamido-2-deoxy-beta-d-glucopyranosyl)-l-serine residue"**

(73) Patentinhaber: **HOECHST AKTIENGESELLSCHAFT**
**Postfach 80 03 20**
**W-6230 Frankfurt am Main 80(DE)**

(72) Erfinder: **König, Wolfgang, Dr.**
**Eppsteiner Strasse 25**
**W-6238 Hofheim am Taunus(DE)**
Erfinder: **Sandow, Jürgen Kurt, Dr.**
**Am Heideplacken 22**
**W-6240 Königstein/Taunus(DE)**
Erfinder: **Kolar, Cenek, Dr.**
**Deutschhausstrasse 20**
**W-3550 Marburg(DE)**

**EP 0 263 521 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Natürlich vorkommende Gonadoliberine (Gn-RH) verschiedener Spezies sind Decapeptide folgender Strukturen:

| | |
|---|---|
| h-,p-,o-Gn-RH | Pgl-His-Trp-Ser-Tyr-Gly-Leu-Arg-Pro-Gly-$NH_2$ |
| g-Gn-RH-I | Pgl-His-Trp-Ser-Tyr-Gly-Leu-Gln-Pro-Gly-$NH_2$ |
| g-Gn-RH-II | Pgl-His-Trp-Ser-His-Gly-Trp-Tyr-Pro-Gly-$NH_2$ |
| sa-Gn-RH | Pgl-His-Trp-Ser-Tyr-Gly Trp-Leu-Pro-Gly-$NH_2$ |
| pe-Gn-RH | Pgl-His-Tyr-Ser-Leu-Glu-Trp-Lys-Pro-Gly-$NH_2$ |

[h- (Mensch), p- (Schwein), o- (Schaf): Biochem. Biophys. Res. Commun. 43 (1971) 1334; g- (Huhn-I): South Africa J. Science 78 (1982) 124; g- (Huhn-II): Proc. Natl. Acad. Sci. USA 81 (1984) 3874; sa- (Lachs): Proc. Natl. Acad. Sci. USA 80 (1983) 2794; pe- (Neunauge): J. Biol. Chem. 261 (1986) 4812-4819.]

Gn-RH wird bei Säugern hauptsächlich im Hypothalamus gebildet und bewirkt in der Hypophyse eine Ausschüttung von Lutropin (LH) und Follitropin (FSH).

Hochwirksame Gn-RH-Agonisten erhält man, wenn Glycin in Position 6 durch hydrophobe D-Aminosäuren und/oder Glycinamid in Position 10 durch Ethylamin ersetzt wird. [M.J. Karten u. J.E. Rivier, Endocrine Reviews 7 (1986) 44-66]. Durch diese Substitutionen werden die Gn-RH-Agonisten schwerer löslich in wäßrigen Lösungen. Bei den Gn-RH-Antagonisten, in denen auch noch die hydrophilen Positionen 1, 2 und 10 gegen hydrophobe Aminosäuren ausgetauscht werden, sinkt die Löslichkeit in Wasser noch stärker. Vor allem für die parenterale und die intranasale Anwendung ist eine gute Wasserlöslichkeit wünschenswert: Der Wirkstoff kann in einem kleineren Volumen appliziert werden. Durch Einführung einer basischen D-Aminosäure (D-Arg, D-Lys, D-Har$(Et)_2$) in Position 6 wurde bei den Gn-RH-Antagonisten die Löslichkeit gesteigert, doch sind diese Derivate nicht gut verträglich, da sie durch die Einführung einer zweiten positiven Ladung in das Molekül eine Histamin-Ausschüttung und Mediator-Freisetzung bewirken.

Es wurde gefunden, daß durch Einführung von D-Serin-O-glycosiden in Position 6 und/oder L-Serin-O-glycosiden in Position 7 die Wasserlöslichkeit von Gn-RH-Analoga beträchtlich verbessert werden konnte, wobei deren biologische Aktivität überraschenderweise sehr gut erhalten blieb.

Die Erfindung betrifft Peptide der allgemeinen Formel I,

```
1 2 3 4   5   6 7 8   9   10
X-A-B-C-Ser-D-E-F-Arg-Pro-G          (I),
```

in welcher

- X Wasserstoff oder $(C_1$-$C_7)$-Acyl bedeutet oder, falls A für Pyroglutamyl steht, abwesend ist;
- A Pgl, dehydro-Pro, Pro, D-Thi, D-Pgl oder gegebenenfalls durch einen oder zwei gleiche oder verschiedene Reste aus der Reihe Brom, Chlor, Fluor, Nitro, Amino, Methyl und Methoxy im Aromaten substituiertes D-Nal(2), in dieser Weise substituiertes D-Phe oder in dieser Weise substituiertes D-Trp bedeutet;
- B His oder gegebenenfalls durch einen oder zwei gleiche oder verschiedene Reste aus der Reihe Brom, Chlor, Fluor, Nitro, Amino, Methyl und Methoxy im Phenylring substituiertes D-Phe bedeutet;
- C Trp, D-Thi, D-Pal(3) oder gegebenenfalls in Position 5 und/oder 6 durch einen oder zwei gleiche oder verschiedene Reste aus der Reihe Brom, Chlor, Fluor, Nitro, Amino, Methyl oder Methoxy substituiertes D-Trp bedeutet;
- D Tyr, Arg oder His bedeutet;
- E D-Ser$(R^1)$, $\beta$-Asn, $\beta$-Asp-OMe, D-Thi oder den Rest einer D-Aminosäure der allgemeinen Formel II bedeutet;

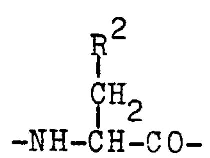

(II)

F Ser($R^1$), Leu, Trp oder Phe bedeutet;
G Gly-$NH_2$, Aza-Gly-$NH_2$, D-Ala-$NH_2$ oder NH-($C_1$-$C_4$)-alkyl, vorzugsweise NH-$C_2H_5$, bedeutet;
$R^1$ einen gegebenenfalls teilgeschützten Glycosylrest mit wenigstens einer freien Hydroxygruppe bedeutet und
$R^2$ Wasserstoff, ($C_1$-$C_4$)-Alkoxycarbonyl, ($C_1$-$C_4$)-Alkoxy-, ($C_1$-$C_4$)-Alkyl, das gegebenenfalls durch ($C_1$-$C_4$)-Alkoxycarbonyl oder ($C_1$-$C_4$)-Alkoxycarbonylamino monosubstituiert ist, Phenyl, das gegebenenfalls durch bis zu drei gleiche oder verschiedene Reste aus der Reihe Chlor, Fluor, Methyl und ($C_1$-$C_4$)-Alkoxy substituiert ist, Naphthyl, 4,5,6,7-Tetrahydrobenzimidazol-2-yl oder Indolyl bedeutet, und deren physiologisch verträglichen Salze, mit der Maßgabe, daß

a) Falls E für einen Rest der Formel II, $\beta$-Asn, $\beta$-Asp-OMe oder D-Thi steht, F ausschließlich die Bedeutung Ser($R^1$) zukommt und
b) falls F für Leu, Phe oder Trp steht, E ausschließlich die Bedeutung D-Ser($R^1$) zukommt.

X in der Bedeutung von ($C_1$-$C_7$)-Acyl steht vorzugsweise für ($C_1$-$C_7$)-Alkanoyl, insbesondere Acetyl, Benzoyl oder ($C_1$-$C_6$)-Alkoxycarbonyl.

Alkylreste können geradkettig oder verzweigt vorliegen.

Falls nicht anders angegeben, werden in Formel I und im folgenden die Dreibuchstabensymbole (vgl. z.B. Pure Appl. Chem. 56 [1984] 595-624, und Eur. J. Biochem. 138 [1984] 9-37) für die Reste der Aminosäuren verwendet. Diesen Symbolen wird das Symbol "D" vorangestellt, wenn es sich um den Rest einer D-Aminosäure handelt; Reste ohne Konfigurationssymbol sind L-konfiguriert.

Schutzgruppen werden in der Literatur üblichen Weise abgekürzt (vgl. z.B. Wünsch et al., Synthese von Peptiden (Houben-Weyl 15/1,2), Stuttgart, Thieme 1974).

Bevorzugt sind Gn-RH-Agonisten der allgemeinen Formel I, in welcher
X abwesend ist;
A Pgl;
B His;
C Trp;
D Tyr oder His;
E D-Ser($R^1$), $\beta$-Asn, $\beta$-Asp-OMe oder den Rest einer D-Aminosäure der Formel II;
F Ser($R^1$), Trp oder Leu und
G Gly-$NH_2$, Aza-Gly-$NH_2$ oder NH-($C_1$-$C_4$)-Alkyl, vorzugsweise -NH-$C_2H_5$,
bedeuten und
$R^1$ und $R^2$ wie oben definiert sind, sowie Gn-RH-Antagonisten der allgemeinen Formel I, in welcher
X Wasserstoff oder ($C_1$-$C_7$)-Acyl bedeutet oder abwesend ist;
A dehydro-Pro, Pro, D-Thi, D-Pgl, gegebenenfalls substituiertes D-Nal(2), gegebenenfalls substituiertes D-Phe oder gegebenenfalls substituiertes D-Trp;
B gegebenenfalls substituiertes D-Phe;
C gegebenenfalls substituiertes D-Trp, D-Thi oder D-Pal(3);
D Tyr, Arg oder His;
E D-Ser($R^1$), D-Thi oder den Rest einer D-Aminosäure der Formel II;
F Ser($R^1$), Leu, Phe oder Trp und
G Gly-$NH_2$, D-Ala-$NH_2$, Aza-Gly-$NH_2$ oder NH-($C_1$-$C_4$)-Alkyl, vorzugsweise NH-$C_2H_5$, bedeuten, und
$R^1$ und $R^2$ wie oben definiert sind.

Besonders bevorzugte Antagonisten sind solche, worin
X ($C_1$-$C_7$)-Acyl, vorzugsweise Acetyl;
A D-Nal(2);
B D-Phe(Cl);
C D-Trp;
D Tyr, His oder Arg;
E D-Ser($R^1$);
F Ser($R^1$), Leu, Phe oder Trp und
G D-Ala-$NH_2$ oder Aza-Gly-$NH_2$ bedeuten.
$R^1$ ist vorzugsweise ein mit in der Kohlenhydratchemie üblichen Schutzgruppen teilgeschützter oder ungeschützter Glycosylrest, der sich von einer Glycopyranose, Glycofuranose oder einem Oligosaccharid ableitet. Es soll zumindest eine Hydroxygruppe ungeschützt sein.

Besonders bevorzugt sind Gn-RH-Analoga mit ungeschützten Glycosylresten. Die Glycosylreste können sowohl $\alpha$- als auch $\beta$-glycosidisch mit dem Serin-Rest verknüpft sein.

$R^1$ kann beispielsweise ein Glucofuranosyl- oder Glucopyranosyl-Rest sein, der sich von natürlich

vorkommenden Aldotetrosen, Aldopentosen, Aldohexosen, Ketopentosen, Ketohexosen, Desoxyaldosen, Aminoaldosen und Oligosacchariden, wie Di- und Trisacchariden, sowie deren Stereoisomeren ableiten.

Diese Glycosylreste $R^1$ leiten sich insbesondere von natürlichen, im Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-mannosamin (ManNAc) oder Disacchariden, wie Maltose (Mal), Lactose (Lac), Cellobiose (Cel), Gentibiose (Gen), N-Acetyl-lactosamin (LacNAc), Chitobiose (Chit), β-Galactopyranosyl-(1-3)-N-acetylgalactosamin und β-Galactopyranosyl-(1-3)- oder -(1-4)-N-acetyl-glucosamin, sowie deren synthetischen Derivaten, wie 2-Desoxy-, 2-Amino-, 2-Acetamido- oder 2-Halogeno-, bevorzugt Bromo- und Jodo-Zucker ab.

Unter den in der Kohlenhydratchemie üblichen Schutzgruppen versteht man z.B. die ($C_1$-$C_{10}$)-Acylschutzgruppen wie ($C_1$-$C_6$)-Alkanoyl (z.B. Acetyl-, Trichloracetyl-, Trifluoracetyl-), Benzoyl- oder p-Nitrobenzoyl-, sowie gegebenenfalls modifizierte Methyl-, Methyloxymethyl-, Benzyl-, Tetrahydropyranyl-, Benzyliden-, Isopropyliden- oder Trityl-Gruppen, wobei hier die Acylschutzgruppen, insbesondere die Acetyl- (Ac-)Gruppe, bevorzugt sind.

Unter physiologisch verträglichen Salzen versteht man insbesondere solche mit anorganischen Säuren, wie HCl, HBr, $H_2SO_4$, $H_3PO_4$, oder organischen Säuren, wie Essigsäure, Maleinsäure, Fumarsäure, Weinsäure, Citronensäure.

Die Erfindung betrifft auch ein Verfahren zur Herstellung von Peptiden der allgemeinen Formel I, das dadurch gekennzeichnet ist, daß man ein Fragment mit N-terminaler freier Aminogruppe kondensiert mit einem Fragment mit C-terminaler freier Carboxylgruppe, eine oder mehrere zum Schutz funktioneller Gruppen gegebenenfalls temporär eingeführte Schutzgruppen abspaltet und das so erhaltene Peptid gegebenenfalls in sein physiologisch verträgliches Salz überführt.

Die Auswahl der Schutzgruppen und die Synthesestrategie wird von der Art und Konfiguration der Aminosäuren sowie der Art der Kupplungsbedingungen bestimmt.

Die Kondensation nach dem erfindungsgemäßen Verfahren erfolgt nach den allgemeinen Methoden der Peptidchemie, bevorzugt nach der Methode der gemischten Anhydride, über Aktivester, Azide oder nach der Carbodiimid-Methode, insbesondere unter Zusatz reaktionsbeschleunigender und racemisierungsverhindernder Substanzen wie z.B. 1-Hydroxy-benzotriazol, N-Hydroxysuccinimid, 3-Hydroxy-4-oxo-3,4-dihydro-1,2,3-benzotriazin, N-Hydroxy-5-norbornen-2,3-dicarboximid, ferner unter Verwendung aktivierter Derivate des 1-Hydroxybenzotriazols oder Anhydriden von Phosphor-Phosphon- und Phosphinsäuren bei einer Reaktionstemperatur zwischen -10 °C und dem Siedepunkt des Reaktionsgemisches, vorzugsweise zwischen -5 °C und 40 °C.

Geeignete Lösungsmittel dafür sind Dimethylformamid, Dimethylacetamid, Hexamethylphosphorsäuretriamid, N-Methylpyrrolidon oder Dimethylsulfoxid. Sofern die Löslichkeit der Komponenten es erlaubt, können auch Lösungsmittel wie Methylenchlorid oder Chloroform eingesetzt werden. Die genannten Methoden sind z.B. in Meienhofer-Gross: "The Peptides", Academic Press, Vol. I, (1979) beschrieben.

Um die Glycosylreste in L- oder D-Serin einzuführen, müssen vorher die Aminogruppe und die Carboxylgruppe geeignet geschützt sein. Als besonders günstig erwiesen sich hierbei Schutzgruppen, die durch katalytische Hydrierung oder durch sekundäre Amine abspaltbar sind. Im ersteren Fall sind dies Schutzgruppen von Benzyl-Typ, wie z.B. der Benzyloxycarbonyl-(Z-) oder p-Nitro-benzyloxycarbonyl-Rest als Aminoschutzgruppe und der Benzyl- (-OBzl) oder p-Nitrobenzylester für die Carboxylgruppe. Mit sekundären Aminen läßt sich der 9-Fluorenyl-methyloxycarbonyl-(Fmoc-)Rest abspalten. Besonders günstig erwies sich die Verwendung von Fmoc-L- bzw. Fmoc-D-Ser-OBzl, da nach Glycosidierung die Benzylester der entsprechenden Fmoc-L-bzw. Fmoc-D-Ser($R^1$)-OBzl durch katalytische Hydrierung unter Erhalt der Fmoc-Gruppe selektiv abgespalten werden konnten. Das ist besonders überraschend, da in letzter Zeit immer wieder berichtet wurde, daß der Fmoc-Rest durch katalytische Hydrierung abgespalten wird [z.B. von R. Geiger und W. König in E. Gross und J. Meienhofer (Eds): The Peptides, Vol. 3, p. 24, Academic Press, 1981].

Bei der Synthese der O-Glycosyl-Serin-Bausteine sind zwei polyfunktionelle Reaktionspartner (Kohlenhydrat und Serin) zu verknüpfen. Beide müssen sich selektiv blockieren und deblockieren lassen. In der Glycosylkomponente muß das anomere Zentrum freisetzbar und funktionalisierbar sein und in der Serinkomponente darf nur die für die Verknüpfung notwendige Hydroxylgruppe deblockiert sein. Je nach dem Typ der angestrebten glycosidischen Bindung (1,2-cis- oder 1,2-trans-Glycoside) ist es notwendig, geeignete Schutzgruppen für die Blockierung der Hydroxy- oder Aminogruppen in die Glycosylkomponente einzuführen sowie Reaktionsbedingungen für den Verknüpfungsschritt auszuarbeiten, der stereoselektiv zu

nur einem der beiden möglichen Anomeren führt.

Für die Herstellung der erfindungsgemäßen Gn-RH-Analoga werden sowohl die in der Literatur bekannten, meist natürlichen Glycosylserin-Bausteine wie z.B. von K. Dill et al. [Carbohydr. Res. 123 (1983) 137-144], H. Kunz [Nach. Chem. Tech. Lab. 32 (1984) 11] und H. Paulsen [Chem. Soc. Res. 13 (1) (1984) 25-45] beschrieben, als auch die artificiellen Glycosylserinderivate, die nach den in der Kohlenhydratchemie üblichen, z. B. von A.F. Bochkov und G.E. Zaikov [Chemistry of the O-glycosidic bond, Pergamon Press 157 (1979)], H. Paulsen [Angew. Chem. 94 (1982) 184-201] und R.R. Schmidt [Angew. Chem. 98 (1986) 213-236] beschriebenen oder modifizierten Glycosidierungsverfahren hergestellt werden, verwendet.

Die erfindungsgemäßen Peptide können unter Benutzung der allgemeinen Methoden der Peptidchemie (Houben-Weyl, Methoden der Organischen Chemie, Band 15/1,2), beispielsweise stufenweise von C-terminalen Ende oder durch Segment-Kondensation hergestellt werden. Besonders bevorzugt wird bei den Gn-RH-Agonisten die Segmentkondensation nach dem Schema

(3-5) + (6-10)
↓
(1-2) + (3-10)
↓
(1-10)

Um bei der Segmentkondensation die mögliche Racemisierung möglichst niedrig zu halten, wird hier bevorzugt mit Dicyclohexylcarbodiimid (DCC) unter Zusatz von 3-Hydroxy-4-oxo-3,4-dihydro-1,2,3-benzotriazin (HOObt) gearbeitet. Als Amino-Schutzgruppen werden bevorzugt der durch katalytische Hydrierung abspaltbare Z-Rest oder der durch sekundäre Amine abspaltbare Fmoc-Rest herangezogen. Der Imidazol-Ring des Histidins wird vorzugsweise durch den 2,4-Dinitrophenyl-(Dnp-)Rest geschützt, der durch Mercaptane oder Hydrazin abgespalten werden kann.

Die erfindungsgemäßen Antagonisten können ebenfalls stufenweise von C-terminalen Ende aus hergestellt werden. Ökonomischer sind aber auch hier Segmentkondensationen, wie z.B.

(1-4) + (5-10) → (1-10).

Bei der Verwendung von Acylgruppen als Schutzgruppen für die Hydroxygruppen des glycosidischen Restes kann man unter Verwendung von Hydrazin bei der Schutzgruppenabspaltung neben völlig ungeschützten Peptiden auch Peptidderivate isolieren, bei denen der Dnp-Rest völlig abgespalten ist, 1-2 Acylgruppen im glycosidischen Rest jedoch noch erhalten sind.

Die Tabelle 1 zeigt die Löslichkeit der synthetisierten Peptide und Glycopeptide in einem gut verträglichen neutralen Puffer, wie er für die nasale Applikation des Protirelins Verwendung findet [Horm. Metab. Res. 15 (1983) 52] und die biologische Wirkung in der Superovulation bei Ratten.

Tabelle 1:

| | $ED_{20-50}$ Super-ovul. (ng/Ratte) | Lösl. in neutralem Nasalpuffer (mg/ml) |
|---|---|---|
| Pgl-His-Trp-Ser-Tyr-D-Ser(tBu)-Leu-Arg-Pro-NH-$C_2H_5$ (= Buserelin) | 3 | 1,25 |
| Pgl-His-Trp-Ser-Tyr-D-Ser(β-D-Glc)-Leu-Arg-Pro-NH-$C_2H_5$ | 12 | 125,0 |
| Pgl-His-Trp-Ser-Tyr-D-Ser(α-D-Man)-Leu-Arg-Pro-NH-$C_2H_5$ | 6 | 62,5 |
| Pgl-His-Trp-Ser-Tyr-D-Ser(β-L-Fuc)-Leu-Arg-Pro-NH-$C_2H_5$ | 6 | 167,0 |
| Pgl-His-Trp-Ser-Tyr-D-Ser(Ac-β-L-Fuc)-Leu-Arg-Pro-NH-$C_2H_5$ | 6 | 10,0 |
| Pgl-His-Trp-Ser-Tyr-D-Ser(β-D-Xyl)-Leu-Arg-Pro-NH-$C_2H_5$ | 6 | 50,0 |
| Pgl-His-Trp-Ser-Tyr-D-Ser(α-L-Rha)-Leu-Arg-Pro-NH-$C_2H_5$ | 3 | 167,0 |
| Pgl-His-Trp-Ser-Tyr-D-Ser(Ac-α-L-Rha)-Leu-Arg-Pro-NH-$C_2H_5$ | 3 | 50,0 |
| Pgl-His-Trp-Ser-Tyr-D-Trp-Leu-Arg-Pro-NH-$C_2H_5$ (LH-RH-T) | 3 | 0,33 |
| Pgl-His-Trp-Ser-Tyr-D-Trp-Ser(β-D-Glc)-Arg-Pro-NH-$C_2H_5$ | 6 | 2,5 |

| | $ED_{20-50}$ Super-ovul. (ng/Ratte) | Lösl. in neutralem Nasalpuffer (mg/ml) |
|---|---|---|
| Pgl-His-Trp-Ser-Tyr-D-Trp-Ser(Ac-β-D-Glc)-Arg-Pro-NH-$C_2H_5$ | 5 | 2,5 |
| Pgl-His-Trp-Ser-Tyr-D-Trp-Ser(β-L-Fuc)-Arg-Pro-NH-$C_2H_5$ | >200 | 2,5 |
| Pgl-His-Trp-Ser-Tyr-D-Trp-Ser(Ac-β-L-Fuc)-Arg-Pro-NH-$C_2H_5$ | 200 | 10,0 |
| Pgl-His-Trp-Ser-Tyr-D-Trp-Ser($Ac_2$-β-L-Fuc)-Arg-Pro-NH-$C_2H_5$ | 200 | 3,3 |
| Pgl-His-Trp-Ser-Tyr-D-Trp-Ser(α-D-Man)-Arg-Pro-NH-$C_2H_5$ | > 24 | 3,3 |
| Pgl-His-Trp-Ser-Tyr-D-Trp-Ser(Ac-α-D-Man)-Arg-Pro-NH-$C_2H_5$ | > 24 | 10,0 |
| Pgl-His-Trp-Ser-Tyr-D-Trp-Ser($Ac_2$-α-D-Man)-Arg-Pro-NH-$C_2H_5$ | > 24 | 10,0 |
| Pgl-His-Trp-Ser-Tyr-D-Trp-Ser(β-D-Xyl)-Arg-Pro-NH-$C_2H_5$ | 6 | 1,7 |
| Pgl-His-Trp-Ser-Tyr-D-Trp-Ser(Ac-β-D-Xyl)-Arg-Pro-NH-$C_2H_5$ | 6 | 2,5 |
| Pgl-His-Trp-Ser-Tyr-D-Trp-Ser(α-L-Rha)-Arg-Pro-NH-$C_2H_5$ | > 24 | 1,4 |
| Pgl-His-Trp-Ser-Tyr-D-Trp-Ser(Ac-α-L-Rha)-Arg-Pro-NH-$C_2H_5$ | >24 | 0,5 |

| | $ED_{20-50}$ Super-ovul. (ng/Ratte) | Lösl. in neutralem Nasalpuffer (mg/ml) |
|---|---|---|
| Pgl-His-Trp-Ser-Tyr-D-Ser(β-L-Xyl)-Leu-Arg-Pro-NH-$C_2H_5$ | 6 | 10 |
| Pgl-His-Trp-Ser-Tyr-D-Ser(β-Lac)-Leu-Arg-Pro-NH-$C_2H_5$ | 12 | 167 |
| Pgl-His-Trp-Ser-Tyr-D-Ser(β-D-Gal)-Leu-Arg-Pro-NH-$C_2H_5$ | 12 | 125 |
| Pgl-His-Trp-Ser-Tyr-D-Ser(α-L-Ara)-Leu-Arg-Pro-NH-$C_2H_5$ | 12 | 125 |
| Pgl-His-Trp-Ser-Tyr-D-Ser(α-D-Ara)-Leu-Arg-Pro-NH-$C_2H_5$ | < 24 | 125 |
| Pgl-His-Trp-Ser-Tyr-D-Ser(β-D-Rib)-Leu-Arg-Pro-NH-$C_2H_5$ | < 6 | 10 |
| Pgl-His-Trp-Ser-Tyr-D-Ser(β-D-GlcNAc)-Leu-Arg-Pro-NH-$C_2H_5$ | < 6 | 125 |
| Pgl-His-Trp-Ser-Tyr-D-Trp-Ser(β-L-Xyl) Arg-Pro-NH-$C_2H_5$ | 48 | 1,1 |
| Pgl-His-Trp-Ser-Tyr-D-Trp-Ser(β-Lac)-Arg-Pro-NH-$C_2H_5$ | <48 | 167 |
| Pgl-His-Trp-Ser-Tyr-D-Trp-Ser(β-D-Gal)-Arg-Pro-NH-$C_2H_5$ | n.d. | 10 |
| Pgl-His-Trp-Ser-Tyr-D-Trp-Ser(α-L-Ara)-Arg-Pro-NH-$C_2H_5$ | 48 | 1,25 |

| | $ED_{20-50}$ Superovul. (ng/Ratte) | Lösl. in neutralem Nasalpuffer (mg/ml) |
|---|---|---|
| Pgl-His-Trp-Ser-Tyr-D-Trp-Ser(α-D-Ara)-Arg-Pro-NH-$C_2H_5$ | 24 | 5 |
| Pgl-His-Trp-Ser-Tyr-D-Trp-Ser(β-L-Rib)-Arg-Pro-NH-$C_2H_5$ | <24 | 25 |
| Pgl-His-Trp-Ser-Tyr-D-Trp-Ser(β-D-GlcNAc)-Arg-Pro-NH-$C_2H_5$ | >24 | 125 |

Aus der Tabelle 1 geht hervor, daß alle Peptide, in denen D-Ser($R^1$) oder Ser($R^1$) eingebaut sind, besser löslich sind als die entsprechenden Vergleichssubstanzen Buserelin oder LH-RH-T. Die biologische Wirkung bleibt bei den Substitutionen von D-Ser($R^1$) in Position 6 sehr gut erhalten, so daß die Relation Löslichkeit zur biologischen Wirkung in allen erfindungsgemäßen Verbindungen größer ist als die des Buserelins. In Position 7 ist sie jedoch auch von der Art des Zuckers abhängig.

Die erfindungsgemäßen Gn-RH-Agonisten wirken bereits in niedriger Dosierung durch Ausschüttung der Gonadotropine fertilitätssteigernd und in wiederholter täglicher hoher Dosierung hemmend auf die Ausschüttung der Gonadotropine und damit kontrazeptiv. Indikationen sind z.B. bei der Frau bei niedriger Dosierung die primäre, aber vorwiegend die sekundäre Amenorrhoe, die Corpus luteum Insuffizienz und beim Mann die Oligospermie. Behandelt werden kann ferner die Pubertas tarda beider Geschlechter und der Kryptorchismus bei Kinderen. In hoher Dosierung wirken die Verbindungen hemmend sowohl auf die Bildung der Gonadotropine als auch von Testosteron und Östrogen und können somit bei Steroid-abhängigen Erkrankungen, wie z.B. bei Prostata- oder Brustkrebs, bei Endometriose oder bei vorzeitigem Eintritt der Geschlechtsreife verwendet werden.

Dosierungen der erfindungsgemäßen Verbindungen, die noch unterhalb der Schwellendosis für die Gonadotropin-Freisetzung liegen, regulieren den Plasma-Parathormon- (PTH-)Spiegel. D.h., daß hoher PTH-Spiegel reduziert und niedriger PTH-Spiegel erhöht wird. Vermutlich über diese PTH-regulierende Wirkung wird auch der Blutzucker beeinflußt, denn PTH stimuliert Glucagon, was wiederum Blutglucose-erhöhend wirkt. Ist Plasma PTH erniedrigt (z.B. durch Glucose), so wirken die Glycopeptide Blutglucose-erhöhend. Bei erhöhtem Plasma-PTH-Spiegel sollten die erfindungsgemäßen Glycopeptide jedoch Blutzucker-erniedrigend wirken. So ist bekannt, daß bei Hyperparathyroidismus (erhöhter Plasma-PTH-Spiegel) der Glucose-Metabolismus und die Insulin-Sensitivität reduziert ist [J. Clin. Endocrinol. Metab. 60 (1985) 229]. Die erfindungsgemäßen Glycopeptide könnten also z.B. bei Hyperparathyroidismus zur Senkung von Plasma-PTH und Blutglucose herangezogen werden. Erhöhtes Plasma PTH findet man auch bei Patienten mit Lebererkrankungen [Clin. Endocr. 19 (1983) 21-28; Acta Endocrinologica 111 (1986) 62-68] und Osteoporose [Horm. metab. Res. 17 (1985) 370-373]. Da diese Verbindungen bereits in Dosen unter der Schwellendosis für die Gonadotropin-Ausschüttung die Gonaden zur Steroidhormon-Synthese anregen [Biochem. J. 232 (1985) 55-59], können sie auch sehr niedrig dosiert bei Östrogen- oder Testosteron-Unterfunktion angewendet werden. Während und nach der Pubertät, sowie vor und nach der Menopause können die erfindungsgemäßen Glycopeptide zur Stimulation der Testosteron- und Östrogen-Synthese herangezogen werden. Sowohl Östrogen als auch Testosteron sind für die Knochenbildung von großer Bedeutung [Clin. Endocrinol. Metab. 9 (1980) 177-205; Acta Endocrinologica 107 (1984) 428-432]. Deshalb sind neben pre- und post-Menopausen-Beschwerden auch Knochenschmerzen und Osteoporose, die auf zu geringe Östrogen- oder Testosteron-Spiegel beruhen, mit den erfindungsgemäßen Peptiden zu behandeln. Da PTH selbst blutdrucksenkend wirkt [Hypertension 5 (1983) Suppl. I, 59-63], kann durch Senkung oder Erhöhung des PTH-Spiegels durch die erfindungsgemäßen Substanzen auch der Blutdruck erhöht oder gesenkt werden. Bei Hyperparathyroidismus ist merkwürdigerweise trotz erhöhtem PTH in etwa 40 % der Fälle der Blutdruck erhöht [Adv. Exp. Med. Biol. 151 (1982) 619]. Das liegt an dem hohen Calcium-Spiegel, denn PTH potenziert den hypertensiven Effekt einer Hypercalcämie [Am. J. Physiol. 250 (1986) F924-F926]. So wirken also die erfindungsgemäßen Glycopeptide bei hypertensivem Hyperparathyroidismus auch blutdrucksenkend.

Die erfindungsgemäßen Gn-RH-Antagonisten wirken hemmend auf die Bildung des Lutropins und Follitropins und damit auch auf die Synthese von Testosteron und Östrogen (s. Tab. 2). Sie können wie hochdosierte Gn-RH-Agonisten bei Gonadotropin-und Steroid-abhängigen Erkrankungen eingesetzt werden.

Tabelle 2: Serum-Testosteron-Spiegel bei männlichen Ratten nach einer Dauerinfusion von 12 µg Peptid/Tag mittels einer Alzey-Minipumpe

| | Serum-Testosteron (ng/ml) |
|---|---|
| unbehandelte Kontrolle | 3,63 ± 0,59 |
| Ac-D-Nal-p-Cl-D-Phe-D-Trp-Ser-His-D-Ser(α-L-Rha)-Leu-Arg-Pro-Azagly-$NH_2$ | 1,34 ± 0,17 |
| Ac-D-Nal-p-Cl-D-Phe-D-Trp-Ser-Tyr-D-Ser(α-L-Rha)-Leu-Arg-Pro-Azagly-$NH_2$ | 2,30 ± 0,47 |
| Ac-D-Nal-p-Cl-D-Phe-D-Trp-Ser-Arg-D-Ser(α-L-Rha)-Leu-Arg-Pro-Azagly-$NH_2$ | 2,27 ± 0,28 |

Die erfindungsgemäßen Verbindungen können intranasal oder parenteral in entsprechender pharmazeutischer Zubereitung verabreicht werden. Für eine intranasale Anwendungform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Als ölige Trägerstoffe oder Lösungsmittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weitere Hilfsstoffe in Lösung, Suspension oder Emulsion gebracht.

Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Die bevorzugten Anwendungsformen beim Menschen sind die intranasale Applikation oder die Verwendung von Implantaten, da die Resorption aus dem Magen-Darm-Trakt nur gering ist und falls eine tägliche parenterale Anwendung für den Patienten unzweckmäßig erscheint.

Mittels eines Dosierzerstäubers werden über eine Spraydüse etwa 0,02-0,2 ml einer Pufferlösung, in der die erforderliche Menge des Wirkstoffs gelöst ist, in die Nase gesprüht. Zur einmaligen Stimulierung der Gonadotropine bewegt man sich bei den Gn-RH-Agonisten im allgemeinen bei einer nasalen Tagesdosis von 25-100 µg/Patient. Beim Kryptorchismus genügen bereits etwa 5-25 µg/Tag und Patient (als Nasallösung verabreicht). Wegen der Langzeitwirkung der Gn-RH-Agonisten können sie zur Stimulierung der Gonadotropine in größeren Intervallen (1-3 Tage) appliziert werden. Für die Hemmung der Gonadotropine und der damit eingestellten Östrogen- und Testosteron-Synthese müssen täglich höhere Dosen an Gn-RH-Agonisten verabreicht werden. Man braucht pro Patient etwa 200-500 µg bei nasaler Anwendung mehrmals täglich. Zur Regulierung des Plasma-PTH-Spiegels und der direkten Stimulierung der Gonaden benötigt man etwa 2,5-10 µg/Tag und Patient. Bei einer parenteralen Applikation können die Dosierungen gegenüber der Intranasal-Dosis um etwa eine Zehnerpotenz gesenkt werden.

Die Einzeldosis in Implantaten zur Steriod-Suppression beträgt beim Menschen 3-8 mg eines Gn-RH-Agonisten für einen Zeitraum von jeweils 4-8 Wochen (Dosisinterval). Als Implantat-Trägermaterial werden vorzugsweise Copolymere der Milchsäure und Glycolsäure, sowie Poly-(3-hydroxybuttersäure) verwendet. Die vorstehenden und folgenden Dosierungen beim Menschen beziehen sich, Kryptorchismus ausgenommen, auf einen normalgewichtigen Erwachsenen von etwa 75 kg Körpergewicht.

In der Veterinärmedizin werden die erfindungsgemäßen Gn-RH-Agonisten vorzugsweise parenteral angewendet. Die Gn-RH-Agonisten können zur Behandlung von acyclischen Tieren und zur Ovulationsinduktion und -synchronisation eingesetzt werden. Die Dosis schwankt je nach Tierart. Empfohlen wird z.B. beim Rind eine Dosis von 10-20 µg, bei der Stute 20-40 µg und beim Kaninchen 0,5-1 µg. Bei Implantaten mit einer Einzel-Dosis von 3-300 µg für einen Zeitraum von 2-4 Wochen kann beim Rind eine Anregung der Gonadenfunktion erreicht werden.

Die erfindungsgemäßen Antagonisten werden beim erwachsenen Menschen intranasal in Dosen von 1-10 mg appliziert. Die Einzeldosis in Implantaten beträgt etwa 5-50 mg für einen Zeitraum von jeweils 4-8 Wochen. Parenteral appliziert, genügen bereits 0,1-1mg.

Weitere verwendete Abkürzungen:

| | |
|---|---|
| HOBt | 1-Hydroxybenzotriazol |
| Nal(2) | 2-Naphthyl-alanin |
| -ONSu | N-Hydroxysuccinimidester |
| Pal(3) | 3-Pyridinyl-alanin |
| Pgl | Pyroglutaminsäure |
| Phe(Cl) | p-Chlor-phenylalanin |
| Thi | 2-Thienylalanin |
| Har | Homo-Arginin |

Die nachstehenden Beispiele sollen die vorliegende Erfindung erläutern, ohne daß diese darauf beschränkt wäre.

Beispiele:

1. Beispiel

Pgl-His-Trp-Ser-Tyr-D-Ser(β-D-Glc)-Leu-Arg-Pro-NH-$C_2H_5$

1a. Z-D-Ser($Ac_4$-β-D-Glc)-OBzl

8,3 g Z-D-Ser-OBzl (24,17 mmol) werden in einer Mischung aus 80 ml Toluol und 80 ml Nitromethan gelöst. Nach Zugabe von 5,16 g (20,24 mmol) $Hg(CN)_2$ wird der Reaktionsansatz auf 60 °C erwärmt und anschließend portionsweise mit 13,0 g (31,42 mmol) 2,3,4,6-Tetra-O-acetyl-α-D-glucopyranosylbromid versetzt. Nach 3 Stunden Reaktionszeit wird der Ansatz analog Beispiel 8a aufgearbeitet.
Ausbeute 16,5 g (87 %)

1b. H-D-Ser($Ac_4$-β-D-Glc)-OH•HCl

9,31 g Z-D-Ser($Ac_4$-β-D-Glc)-OBzl werden in einer Mischung aus 100 ml Essigsäureethylester und 100 ml Methanol gelöst und in Gegenwart von 9,3 g Palladium/Kohle (10%ig) unter gleichzeitiger Zugabe von methanolischer HCl-Lösung (1,125 g HCl) 3 Stunden hydriert. Nach dem Abfiltrieren des Katalysators und Nachwaschen mit Methanol wird die Lösung im Vakuum eingedampft. Der kristalline Rückstand wird aus Essigsäureethylester umkristallisiert.

Ausbeute 6,18 g (87,1 %); $[\alpha]_D^{20}$ = - 32,5° (c = 1, in Wasser).

1c. Z-D-Ser($Ac_4$-β-D-Glc)-OH

Zu einer Lösung von 5,08 g (10 mmol) H-D-Ser($Ac_4$-β-D-Glc)-OH•HCl in einer Mischung aus 15 ml Dimethylformamid und 15 ml Wasser gibt man 2,6 ml N-Äthylmorpholin und 3 g Z-ONSu. Man läßt etwa 24 Stunden bei Raumtemperatur rühren. Anschließend wird mit 25 ml 1N HCl angesäuert und mit Wasser verdünnt. Der Niederschlag wird abgesaugt und aus Essigester/Petrolether umkristallisiert.

Ausbeute 5,93 g (97,8 %); Schmp. 159-162° C, $[\alpha]_D^{24}$ = -37,1° (c=1, in Methanol)

1d. Z-D-Ser($Ac_4$-β-D-Glc)-Leu-Arg-Pro-NH-$C_2H_5$-tosylat

Zu einer Lösung von 1,82 g (3 mmol) Z-D-Ser($Ac_4$-β-D-Glc)-OH, 2,27 g H-Leu-Arg-Pro-NH-$C_2H_5$-ditosylat und 0,4 g HOBt in 10 ml Dimethylformamid gibt man bei 0° C 0,4 ml N-Ethylmorpholin und 660 mg DCC. Man läßt eine Stunde bei 0° C rühren und anschließend bei Raumtemperatur stehen. Nach etwa 24 Stunden wird der Niederschlag abgesaugt und das Filtrat eingeengt. Der Rückstand wird in n-Pentanol gelöst und nacheinander zweimal mit NaCl-haltigem Wasser und dreimal mit gesättigter wäßriger $NaHCO_3$-Lösung ausgeschüttelt. Die organische Phase wird eingeengt und der Rückstand warm in Essigester gelöst. Nun wird mit Diethylether die Substanz ausgefällt. Der Niederschlag wird abgesaugt und mit Ether gewaschen.

Ausbeute 2,49 g (83 %), Schmp. 104-112° C (unter Zersetzung), $[\alpha]_D^{24}$ = -43° (c=1, in Methanol).

1e. Z-Trp-Ser-Tyr-D-Ser($Ac_4$-β-D-Glc)-Leu-Arg-Pro-NH-$C_2H_5$•HCl

2,3 g (2,3 mmol) Z-D-Ser($Ac_4$-β-D-Glc)-Leu-Arg-Pro-NH-$C_2H_5$-tosylat werden in Methanol am Autotitrator (Zugabe von 1N methanolischer HCl) bei pH 4,5 unter Zusatz von Pd-Katalysator katalytisch mit Wasserstoff hydriert. Nach beendigter Reaktion wird vom Katalysator abgesaugt und das Filtrat eingeengt. Der Rückstand wird mit Essigester verrieben.

Ausbeute 1,44 g (67 %).

1,4 g der oben gewonnenen Substanz (1,5 mmol H-D-Ser($Ac_4$-β-D-Glc)-Leu-Arg-Pro-NH-$C_2H_5$•2HCl) werden zusammen mit 0,88 g Z-Trp-Ser-Tyr-OH und 0,244 g HOObt in 6 ml Dimethylformamid gelöst. Dazu gibt man bei 0° C 0,2 ml N-Ethylmorpholin und 330 mg DCC. Man verfährt nun analog Beispiel 1d. Die Substanz ist in Essigester unlöslich und wird deshalb lediglich mit Essigester verrieben. Zur Reinigung löst man die Substanz in Methanol, gibt methanolische HCl bis zu schwach sauren Reaktion hinzu und fällt mit Essigester.

Ausbeute 1,3 g, amorphe Substanz, $[\alpha]_D^{22}$ = -42° (c=1, in Methanol).

1f. H-Trp-Ser-Tyr-D-Ser($Ac_4$-β-D-Glc)-Leu-Arg-Pro-NH-$C_2H_5$•2HCl

1,2 g Z-Trp-Ser-Tyr-D-Ser($Ac_4$-β-D-Glc)-Leu-Arg-Pro-NH-$C_2H_5$•HCl werden analog Beispiel 1e katalytisch hydriert.

Ausbeute 1,06 g; $[\alpha]_D^{22}$ = -36,3° (c=1, in Methanol).

1g. Pgl-His-Trp-Ser-Tyr-D-Ser(β-D-Glc)-Leu-Arg-Pro-NH-$C_2H_5$-acetat

Zu einer Lösung von 0,55 g (0,5 mmol) H-Trp-Ser-Tyr-D-Ser($Ac_4$-β-D-Glc)-Leu-Arg-Pro-NH-$C_2H_5$•2HCl, 0,22 g Pgl-His(Dnp)-OH und 81 mg HOObt in 3 ml Dimethylacetamid gibt man bei 0° C 0,065 ml N-Ethylmorpholin und 0,11 g DCC. Man rührt eine Stunde bei 0° C und läßt anschließend bei Raumtemperatur stehen. Nach etwa 20 Stunden gibt man 0,25 ml Hydrazinhydrat zu und läßt vier Stunden rühren. Der Niederschlag wird abgesaugt. Das Filtrat läßt man in etwa 100 ml Essigsäureethylester eintropfen. Der Niederschlag wird abgesaugt und aus Methanol/Essigester umgefällt. Der ausgefallene Niederschlag wird abgesaugt und mit Essigester gewaschen. Zur Umsalzung in das Acetat wird die Verbindung in Wasser gelöst und mit einem schwach basischen Ionenaustauscher in der Acetat-Form verrührt. Der Austaucher wird abgesaugt und das Filtrat gefriergetrocknet.

Ausbeute 500 mg.

Gereinigt wird an alkylierten Dextrangelen.

Ausbeute 210 mg; $[\alpha]_D^{26}$ = -42,3° (c=1, in Wasser).

2. Beispiel

Pgl-His-Trp-Ser-Tyr-D-Ser(α-D-Man)-Leu-Arg-Pro-NH-$C_2H_5$

2a. Z-D-Ser($Ac_4$-α-D-Man)-OBzl

Analog Beispiel 1a werden Z-D-Ser-OBzl mit 2,3,4,6-Tetra-O-acetyl-α-D-mannopyranosylbromid umgesetzt.

2b. H-D-Ser($Ac_4$-α-D-Man)-OH•HCl

Z-D-Ser($Ac_4$-α-D-Man)-OBzl werden analog Beispiel 1b katalytisch hydriert.
$[\alpha]_D^{20}$ = +42,3° (c=1, in Wasser).

2c. Z-D-Ser($Ac_4$-α-D-Man)-OH

4,97 g (9 mmol) H-D-Ser($Ac_4$-α-D-Man)-OH•HCl werden analog Beispiel 1c mit 2,34 g N-Ethylmorpholin und 2,7 g Z-ONSu umgesetzt. Der Ansatz wird eingeengt und der Rückstand zwischen Wasser (mit $KHSO_4$ auf pH 3 angesäuert) und Essigester verteilt. Die Essigesterphase wird über $Na_2SO_4$ getrocknet und eingeengt. Es bleiben 4,9 g (89 %) eines Öls zurück. Die Substanz kristallisierte nicht.

2d. Z-D-Ser($Ac_4$-α-D-Man)-Leu-Arg-Pro-NH-$C_2H_5$

4,9 g (8 mmol) Z-D-Ser($Ac_4$-α-D-Man)-OH werden analog Beispiel 1 d mit 6,05 g H-Leu-Arg-Pro-NH-$C_2H_5$-ditosylat umgesetzt. Die Substanz wird aus Essigester/Ether gefällt.
Ausbeute 5,55 g, Schmp. 96-102° C (unter Zersetzung), $[\alpha]_D^{24}$ = -23,5° (c=1, in Methanol).

2e. H-D-Ser($Ac_4$-α-D-Man)-Leu-Arg-Pro-NH-$C_2H_5$•2HCl

5 g Z-D-Ser($Ac_4$-α-D-Man)-Leu-Arg-Pro-NH-$C_2H_5$ werden analog Beispiel 1e katalytisch hydriert.
Ausbeute 3,45 g; Schmp. 55-66° C (unter Zersetzung), $[\alpha]_D^{24}$ = -26,4° (c=1, in Methanol).

2f. Z-Trp-Ser-Tyr-D-Ser($Ac_4$-α-D-Man)-Leu-Arg-Pro-NH-$C_2H_5$

2,81 g (3 mmol) H-D-Ser($Ac_4$-α-D-Man)-Leu-Arg-Pro-NH-$C_2H_5$•2HCl werden analog Beispiel 1e mit 1,76 g Z-Trp-Ser-Tyr-OH umgesetzt.
Ausbeute 3,45 g, amorph; $[\alpha]_D^{23}$ = -22,1° (c=1, in Methanol).

2g. H-Trp-Ser-Tyr-D-Ser($Ac_4$-α-D-Man)-Leu-Arg-Pro-NH-$C_2H_5$•2HCl

3 g Z-Trp-Ser-Tyr-D-Ser($Ac_4$-α-D-Man)-Leu-Arg-Pro-NH-$C_2H_5$ werden analog Beispiel 1e katalytisch hydriert.
Ausbeute 2,4 g; Schmp. 160-162° C (unter Zersetzung); $[\alpha]_D^{23}$ = -20,9° (c=1, in Methanol).

2h. Pgl-His-Trp-Ser-Tyr-D-Ser(α-D-Man)-Leu-Arg-Pro-NH-$C_2H_5$-acetat

1,1 g (1 mmol) H-Trp-Ser-Tyr-D-Ser($Ac_4$-α-D-Man)-Leu-Arg-Pro-NH-$C_2H_5$•2HCl werden analog Beispiel 1g mit 0,45 g Pgl-His(Dnp)-OH umgesetzt.
Rohausbeute als Acetat: 870 mg.
Ausbeute nach chromatographischer Reinigung: 535 mg, amorph; $[\alpha]_D^{22}$ = -31,4° (c=1, in Wasser)

3. Beispiel

Pgl-His-Trp-Ser-Tyr-D-Ser(β-L-Fuc)-Leu-Arg-Pro-NH-$C_2H_5$ und Pgl-His-Trp-Ser-Tyr-D-Ser(Ac-β-L-Fuc)-Leu-Arg-Pro-NH-$C_2H_5$

3a. Fmoc-D-Ser($Ac_3$-β-L-Fuc)-OBzl

Analog Beispiel 8a werden Fmoc-D-Ser-OBzl mit 2,3,4-Tri-O-acetyl-α-L-fucopyranosylbromid miteinander umgesetzt.

3b. Fmoc-D-Ser($Ac_3$-β-L-Fuc)-OH

Fmoc-D-Ser($Ac_3$-β-L-Fuc)-OBzl werden analog Beispiel 8b katalytisch hydriert.
$[\alpha]_D^{20}$ = -0,4° (c=1, in Essigsäureethylester).

3c. Fmoc-D-Ser($Ac_3$-β-L-Fuc)-Leu-Arg-Pro-NH-$C_2H_5$-tosylat

4,19 g (7 mmol) Fmoc-D-Ser-($Ac_3$-β-L-Fuc)-OH wird analog Beispiel 1d mit 5,29 g H-Leu-Arg-Pro-NH-$C_2H_5$-ditosylat umgesetzt. Die Substanz wird aus Essigester/Ether gefällt.
Ausbeute 6,87 g, amorphe Substanz; $[\alpha]_D^{23}$ = -30,6° (c=1, in Methanol).

3d. H-D-Ser($Ac_3$-β-L-Fuc)-Leu-Arg-Pro-NH-$C_2H_5$-tosylat

5,9 g (ca. 5 mmol) Fmoc-D-Ser($Ac_3$-β-L-Fuc)-Leu-Arg-Pro-NH-$C_2H_5$-tosylat werden in 10 ml Dimethylformamid gelöst. Dazu gibt man 0,5 ml (50 mmol) Diethylamin und läßt 1,5 Stunden stehen, engt ein und verreibt mit Ether.
Ausbeute 4,6 g, amorphe Substanz; $[\alpha]_D^{23}$ = -39,3° (c=1, in Methanol).

3e. Z-Trp-Ser-Tyr-D-Ser($Ac_3$-β-L-Fuc)-Leu-Arg-Pro-NH-$C_2H_5$-tosylat

Zu einer Lösung von 3,84 g (4 mmol) H-D-Ser($Ac_3$-β-L-Fuc)-Leu-Arg-Pro-NH-$C_2H_5$-tosylat, 2,35 g Z-Trp-Ser-Tyr-OH und 0,652 g HOObt in 20 ml Dimethylformamid gibt man bei 0°C 0,88 g DCC. Man läßt eine Stunde bei 0°C rühren und anschließend auf Raumtemperatur kommen. Nach etwa 24 Stunden wird der Niederschlag abgesaugt und das Filtrat eingeengt. Der Rückstand wird mit Essigester verrieben, abgesaugt und aus Methanol/Ether umgefällt.
Ausbeute 5,58 g, amorph; $[\alpha]_D^{23}$ = -28,6° (c=1, in Methanol).

3f. H-Trp-Ser-Tyr-D-Ser($Ac_3$-β-L-Fuc)-Leu-Arg-Pro-NH-$C_2H_5$-tosylat•HCl

4,6 g Z-Trp-Ser-Tyr-D-Ser($Ac_3$-β-L-Fuc)-Leu-Arg-Pro-NH-$C_2H_5$-tosylat werden analog Beispiel 1e katalytisch hydriert.
Ausbeute 3,94 g, amorph; $[\alpha]_D^{23}$ = -24° (c=1, in Methanol).

3g. Pgl-His-Trp-Ser-Tyr-D-Ser(β-L-Fuc)-Leu-Arg-Pro-NH-$C_2H_5$-acetat und Pgl-His-Trp-Ser-Tyr-D-Ser(Ac-β-L-Fuc)-Leu-Arg-Pro-NH-$C_2H_5$-acetat.

1,28 g H-Trp-Ser-Tyr-D-Ser($Ac_3$-β-L-Fuc)-Leu-Arg-Pro-NH-$C_2H_5$-tosylat•HCl und 0,45 g Pgl-His(Dnp)-OH (1 mmol) werden analog Beispiel 1g umgesetzt.
Ausbeute an Rohsubstanz-acetat: 825 mg.
Nach der chromatographischen Reinigung erhält man zwei Fraktionen und eine Mischfraktion:
1. Fraktion: 181 mg Pgl-His-Trp-Ser-Tyr-D-Ser(Ac-β-L-Fuc)-Leu-Arg-Pro-NH-$C_2H_5$-acetat, $[\alpha]_D^{26}$ = -33,7° (c=1, in Wasser);
2. Fraktion: 211 mg Pgl-His-Trp-Ser-Tyr-D-Ser-(β-L-Fuc)-Leu-Arg-Pro-NH-$C_2H_5$-acetat; $[\alpha]_D^{26}$ = -36,8° (c=1, in Wasser);
Mischfraktion der beiden Verbindungen: 277 mg.

4. Beispiel:

Pgl-His-Trp-Ser-Tyr-D-Ser(β-D-Xyl)-Leu-Arg-Pro-NH-$C_2H_5$

4a. Fmoc-D-Ser($Ac_3$-β-D-Xyl)-OBzl

Analog Beispiel 8a werden Fmoc-D-Ser-OBzl mit 2,3,4-Tri-O-acetyl-α-D-xylopyranosylbromid umgesetzt.

4b. Fmoc-D-Ser($Ac_3$-β-D-Xyl)-OH

Fmoc-D-Ser($Ac_3$-β-D-Xyl)-OBzl werden analog Beispiel 8b katalytisch hydriert.
$[\alpha]_D^{20}$ = -46,8° (c=1, in Essigsäureethylester).

4c. H-D-Ser($Ac_3$-β-D-Xyl)-Leu-Arg-Pro-NH-$C_2H_5$-tosylat

4,1 g (7 mmol) Fmoc-D-Ser($Ac_3$-β-D-Xyl)-OH werden analog Beispiel 1d mit 5,3 g H-Leu-Arg-Pro-NH-$C_2H_5$-ditosylat umgesetzt und der schaumige Rückstand analog Beispiel 3d in 20 ml Dimethylformamid mit 1,4 ml Diethylamin behandelt.

Ausbeute 7 g.

Zur Reinigung wird die Substanz zwischen Essigester und Wasser verteilt. Die wäßrige Phase wird gefriergetrocknet.

Ausbeute 5,28 g, amorph; $[\alpha]_D^{23}$ = -71,6° (c = 1, in Wasser).

4d. Z-Trp-Ser-Tyr-D-Ser($Ac_3$-β-D-Xyl)-Leu-Arg-Pro-NH-$C_2H_5$-tosylat

2,8 g (3 mmol) H-D-Ser($Ac_3$-β-D-Xyl)-Leu-Arg-Pro-NH-$C_2H_5$-tosylat werden analog Beispiel 3e mit 1,78 g Z-Trp-Ser-Tyr-OH umgesetzt.

Ausbeute 2,9 g, amorph; $[\alpha]_D^{23}$ = -47,5° (c = 1, in Methanol).

4e. H-Trp-Ser-Tyr-D-Ser($Ac_3$-β-D-Xyl)-Leu-Arg-Pro-NH-$C_2H_5$-ditosylat

2,0 g Z-Trp-Ser-Tyr-D-Ser($Ac_3$-β-D-Xyl)-Leu-Arg-Pro-NH-$C_2H_5$-tosylat werden in 35 ml Methanol (Zugabe von 1N methanolischer p-Toluolsulfonsäure) bei pH 4,5 unter Zugabe von Pd-Katalysator katalytisch mit Wasserstoff hydriert. Nach beendigter Reaktion wird der Katalysator abgesaugt, das Filtrat eingeengt und der Rückstand mit Essigester verrieben. Der Niederschlag wird abgesaugt und getrocknet.

Ausbeute 1,64 g, amorph; $[\alpha]_D^{23}$ = -42,1° (c = 1, in Methanol).

4f. Pgl-His-Trp-Ser-Tyr-D-Ser(β-D-Xyl)-Leu-Arg-Pro-NH-$C_2H_5$-acetat

1,54 g (1 mmol) H-Trp-Ser-Tyr-D-Ser($Ac_3$-β-D-Xyl)-Leu-Arg-Pro-NH-$C_2H_5$-ditosylat werden analog Beispiel 1g mit 0,45 g Pgl-His(Dnp)-OH umgesetzt.

Ausbeute als Rohsubstanz-acetat: 1,03 g.

Ausbeute nach chromatographischer Reinigung: 737,3 mg; $[\alpha]_D^{26}$ = -46,5° (c = 1, in Wasser).

5. Beispiel:

Pgl-His-Trp-Ser-Tyr-D-Ser(α-L-Rha)-Leu-Arg-Pro-NH-$C_2H_5$ und Pgl-His-Trp-Ser-Tyr-D-Ser(Ac-α-L-Rha)-Leu-Arg-Pro-NH-$C_2H_5$

5a. Fmoc-D-Ser($Ac_3$-α-L-Rha)-OBzl

Analog Beispiel 8a werden Fmoc-D-Ser-OBzl mit 2,3,4-Tri-O-acetyl-α-L-rhamnopyranosylbromid umgesetzt.

5b. Fmoc-D-Ser($Ac_3$-α-L-Rha)-OH

Fmoc-D-Ser($Ac_3$-α-L-Rha)-OBzl werden analog Beispiel 8b katalytisch hydriert.

$[\alpha]_D^{20}$ = -37,9° (c = 1, in Essigsäureethylester)

5c. H-D-Ser($Ac_3$-α-L-Rha)-Leu-Arg-Pro-NH-$C_2H_5$-tosylat

4,2 g (7mmol) Fmoc-D-Ser($Ac_3$-α-L-Rha)-OH werden analog Beispiel 1d mit 5,3 g H-Leu-Arg-Pro-NH-$C_2H_5$-ditosylat umgesetzt.

Der schaumige Rückstand wird analog Beispiel 3d und 4e umgesetzt und gereinigt.

Ausbeute 5,52 g, amorph; $[\alpha]_D^{26}$ = -77,6° (c = 1, in Wasser).

5d. Z-Trp-Ser-Tyr-D-Ser($Ac_3$-α-L-Rha)-Leu-Arg-Pro-NH-$C_2H_5$-tosylat

3,8 g (4 mmol) H-D-Ser($Ac_3$-α-L-Rha)-Leu-Arg-Pro-NH-$C_2H_5$-tosylat werden analog Beispiel 3e mit 2,36 g Z-Trp-Tyr-OH umgesetzt.

Ausbeute 5,2 g, amorph; $[\alpha]_D^{26}$ = -49,2° (c = 1, in Methanol).

5e. H-Trp-Ser-Tyr-D-Ser($Ac_3$-α-L-Rha)-Leu-Arg-Pro-NH-$C_2H_5$-ditosylat

4,54 g (3 mmol) Z-Trp-Ser-Tyr-D-Ser($Ac_3$-α-L-Rha)-Leu-Arg-Pro-NH-$C_2H_5$-tosylat werden analog Beispiel 4e katalytisch hydriert.
Ausbeute 4,2 g, amorph; $[\alpha]_D^{26}$ = -46,7° (c=1, in Methanol).

5f. Pgl-His-Trp-Ser-Tyr-D-Ser(α-L-Rha)-Leu-Arg-Pro-NH-$C_2H_5$-acetat und Pgl-His-Trp-Ser-Tyr-D-Ser(Ac-α-L-Rha)-Leu-Arg-Pro-NH-$C_2H_5$-acetat

1,55 g (1 mmol) H-Trp-Ser-Tyr-D-Ser($Ac_3$-α-L-Rha)-Leu-Arg-Pro-NH-$C_2H_5$-ditosylat werden analog Beispiel 1g mit 0,45 g Pgl-His(Dnp)-OH umgesetzt.
Ausbeute an Rohsubstanz-acetat: 1,056 g.
Nach chromatographischer Reinigung:
1. Fraktion: 378 mg Pgl-His-Trp-Ser-Tyr-D-Ser(Ac-α-L-Rha)-Leu-Arg-Pro-NH-$C_2H_5$-acetat; $[\alpha]_D^{25}$ = -46,8° (c=1, in Wasser);
2. Fraktion: 287 mg Pgl-His-Trp-Ser-Tyr-D-Ser(α-L-Rha)-Leu-Arg-Pro-NH-$C_2H_5$-acetat; $[\alpha]_D^{25}$ = -51,1° (c=1, in Wasser);
Mischfraktion: 117,1 mg.

Beispiel 6:

Pgl-His-Trp-Ser-Tyr-D-Trp-Ser(β-D-Glc)-Arg-Pro-NH-$C_2H_5$ und Pgl-His-Trp-Ser-Tyr-D-Tyr-Ser(Ac-β-D-Glc)-Arg-Pro-NH-$C_2H_5$

6a. Fmoc-Ser($Ac_4$-β-D-Glc)-OBzl

Analog Beispiel 8a werden Fmoc-Ser-OBzl mit 2,3,4,6-Tetra-O-acetyl-α-D-glucopyranosylbromid umgesetzt.

6b. Fmoc-Ser($Ac_4$-β-D-Glc)-OH

Fmoc-Ser($Ac_4$-β-D-Glc)-OBzl werden analog Beispiel 8b katalytisch hydriert.
$[\alpha]_D^{20}$ = -6,1° (c=1, in Essigsäureethyleester).

6c. H-Ser($Ac_4$-β-D-Glc)-Arg-Pro-NH-$C_2H_5$-tosylat

4,6 g (7 mmol) Fmoc-Ser($Ac_4$-β-D-Glc)-OH werden analog Beispiel 1d mit 4,5 g H-Arg-Pro-NH-$C_2H_5$-ditosylat umgesetzt. Der schaumige Rückstand wird analog Beispiel 3d und 4c umgesetzt. Man muß hier jedoch länger reagieren lassen (3 Stunden).
Ausbeute 3,25 g

6d. Fmoc-D-Trp-Ser($Ac_4$-β-D-Glc)-Arg-Pro-NH-$C_2H_5$-tosylat

Zu einer Lösung von 1,5 g Fmoc-D-Trp-OH (3,5 mmol), 3,6 g H-Ser($Ac_4$-β-D-Glc)-Arg-Pro-NH-$C_2H_5$-tosylat und 0,47 g HOBt in 20 ml Dimethylformamid gibt man bei 0° C 770 mg DCC, läßt 1 Stunde bei 0° C rühren und dann auf Raumtemperatur kommen. Nach etwa 24 Stunden wird der Niederschlag abgesaugt und das Filtrat eingeengt. Der Rückstand wird zwischen n-Pentanol und Wasser verteilt. Die organische Phase wird anschließend nacheinander mit gesättigter wäßriger $NaHCO_3$-Lösung und Wasser ausgeschüttelt und eingeengt. Der Rückstand wird mit Petroläther verrieben, abgesaugt und getrocknet.
Ausbeute 4,38 g, amorph; $[\alpha]_D^{23}$ = -29,0° (c=1, in Methanol).

6e. H-D-Trp-Ser($Ac_4$-β-D-Glc)-Arg-Pro-NH-$C_2H_5$-tosylat

4 g Fmoc-D-Trp-Ser($Ac_4$-β-D-Glc)-Arg-Pro-NH-$C_2H_5$-tosylat werden analog Beispiel 3d und 4c umgesetzt.
Ausbeute 2,5 g; $[\alpha]_D^{25}$ = -69,2° (c=1, in Wasser).

6f. Z-Trp-Ser-Tyr-D-Trp-Ser($Ac_4$-β-D-Glc)-Arg-Pro-NH-$C_2H_5$-tosylat

1,18 g (2 mmol) Z-Trp-Ser-Tyr-OH werden analog Beispiel 3e mit 1,8 g H-D-Trp-Ser($Ac_4$-β-D-Glc)-Arg-Pro-NH-$C_2H_5$-tosylat umgesetzt. Die Substanz wird nun aus n-Pentanol/Ether umgefällt.
Ausbeute 2,35 g, amorph; $[\alpha]_D^{25}$ = -33,4° (c=1, in Methanol).

6g. H-Trp-Ser-Tyr-D-Trp-Ser($Ac_4$-β-D-Glc)-Arg-Pro-NH-$C_2H_5$-tosylat•HCl

2,2 g Z-Trp-Ser-Tyr-D-Trp-Ser($Ac_4$-β-D-Glc)-Arg-Pro-NH-$C_2H_5$-tosylat werden analog Beispiel 1e katalytisch hydriert.
Ausbeute 1,78 g, amorph; $[\alpha]_D^{23}$ = - 30,0° (c=1, in Methanol).

6h. Pgl-His-Trp-Ser-Tyr-D-Trp-Ser(β-D-Glc)-Arg-Pro-NH-$C_2H_5$-acetat und Pgl-His-Trp-Ser-Tyr-D-Trp-Ser(Ac-β-D-Glc)-Arg-Pro-NH-$C_2H_5$-acetat

0,45 g (1 mmol) Pgl-His(Dnp)-OH werden analog Beispiel 1g mit 1,43 g H-Trp-Ser-Tyr-D-Trp-Ser($Ac_4$-β-D-Glc)-Arg-Pro-NH-$C_2H_5$-tosylat•HCl umgesetzt.
Ausbeute an Rohsubstanz-acetat: 939 mg.
Nach chromatographischer Reinigung:
1. Fraktion: 332 mg Pgl-His-Trp-Ser-Tyr-D-Trp-Ser(β-D-Glc)-Arg-Pro-NH-$C_2H_5$-acetat; $[\alpha]_D^{26}$ = -56,7° (c=1, in Wasser);
2. Fraktion: 112 mg Pgl-His-Trp-Ser-Tyr-D-Trp-Ser(Ac-β-D-Glc)-Arg-Pro-NH-$C_2H_5$-acetat; $[\alpha]_D^{26}$ = -50,4° c=1, in Wasser);
Mischfraktion: 217 mg.

7. Beispiel:

Pgl-His-Trp-Ser-Tyr-D-Trp-Ser(β-L-Fuc)-Arg-Pro-NH-$C_2H_5$,
Pgl-His-Trp-Ser-Tyr-D-Trp-Ser(Ac-β-L-Fuc)-Arg-Pro-NH-$C_2H_5$ und
Pgl-His-Trp-Ser-Tyr-D-Trp-Ser($Ac_2$-β-L-Fuc)-Arg-Pro-NH-$C_2H_5$

7a. Fmoc-Ser($Ac_3$-β-L-Fuc)-OBzl

Analog Beispiel 8a werden Fmoc-Ser-OBzl mit 2,3,4-Tri-O-acetyl-α-L-fucopyranosylbromid umgesetzt.

7b. Fmoc-Ser($Ac_3$-β-L-Fuc)-OH

Fmoc-Ser($Ac_3$-β-L-Fuc)-OBzl wird analog Beispiel 8b katalytisch hydriert.
$[\alpha]_D^{20}$ = +17,4° (c=1, in Essigsäureethylester).

7c. H-Ser($Ac_3$-β-L-Fuc)-Arg-Pro-NH-$C_2H_5$-tosylat

4,2 g (7 mmol) Fmoc-Ser($Ac_3$-β-L-Fuc)-OH werden analog Beispiel 1d mit 4,5 g H-Arg-Pro-NH-$C_2H_5$-ditosylat umgesetzt. Der resultierende Schaum wird analog Beispiel 6c in 20 ml Dimethylformamid mit 0,7 ml Diethylamin umgesetzt.
Ausbeute 3,07 g; $[\alpha]_D^{25}$ = -22,8° (c=1, in Wasser).

7d. Fmoc-D-Trp-Ser($Ac_3$-β-L-Fuc)-Arg-Pro-NH-$C_2H_5$-tosylat

1,5 g (3,5 mmol) Fmoc-D-Trp-OH werden analog Beispiel 6d mit 2,9 g H-Ser($Ac_3$-β-L-Fuc)-Arg-Pro-NH-$C_2H_5$-tosylat umgesetzt.
Ausbeute 3,65 g, amorph; $[\alpha]_D^{22}$ = -12,8° (c=1, Methanol).

7e. H-D-Trp-Ser($Ac_3$-β-L-Fuc)-Arg-Pro-NH-$C_2H_5$-tosylat

3,5 g (2,8 mmol) Fmoc-D-Trp-Ser($Ac_3$-β-L-Fuc)-Arg-Pro-NH-$C_2H_5$-tosylat werden analog Beispiel 3d und 4c umgesetzt.
Ausbeute 1,81 g, amorph; $[\alpha]_D^{22}$ = -48,2° (c=1, in Wasser).

7f. Z-Trp-Ser-Tyr-D-Trp-Ser($Ac_3$-β-L-Fuc)-Arg-Pro-NH-$C_2H_5$-tosylat

0,89 g (1,5 mmol) Z-Trp-Ser-Tyr-OH werden analog Beispiel 3e mit 1,52 g H-D-Trp-Ser($Ac_3$-$\beta$-L-Fuc)-Arg-Pro-NH-$C_2H_5$-tosylat umgesetzt.

Ausbeute 2,1 g, amorph; $[\alpha]_D^{22}$ = -28,5° (c=1, in Methanol).

7g. H-Trp-Ser-Tyr-D-Trp-Ser($Ac_3$-$\beta$-L-Fuc)-Arg-Pro-NH-$C_2H_5$-ditosylat

1,9 g (1,2 mmol) Z-Trp-Ser-Tyr-D-Trp-Ser($Ac_3$-$\beta$-L-Fuc)-Arg-Pro-NH-$C_2H_5$-tosylat werden analog Beispiel 4e katalytisch hydriert.

Ausbeute 1,71 g, amorph; $[\alpha]_D^{22}$ = -45,6° (c=1, in Wasser).

7h. Pgl-His-Trp-Ser-Tyr-D-Trp-Ser($\beta$-L-Fuc)-Arg-Pro-NH-$C_2H_5$-acetat, Pgl-His-Trp-Ser-Tyr-D-Trp-Ser(Ac-$\beta$-L-Fuc)-Arg-Pro-NH-$C_2H_5$-acetat und Pgl-His-Trp-Ser-Tyr-D-Trp-Ser($Ac_2$-$\beta$L-Fuc)-Arg-Pro-NH-$C_2H_5$-acetat

0,45 g (1 mmol) Pgl-His(Dnp)-OH werden analog Beispiel 1g mit 1,62 g H-Trp-Ser-Tyr-D-Trp-Ser($Ac_3$-$\beta$-L-Fuc)-Arg-Pro-NH-$C_2H_5$-ditosylat umgesetzt.

Ausbeute an Rohsubstanz-acetat: 1,23 g.

Nach chromatographischer Reinigung:

1. Fraktion: 111,6 mg Pgl-His-Trp-Ser-Tyr-D-Trp-Ser($Ac_2$-$\beta$-L-Fuc)-Arg-Pro-NH-$C_2H_5$-acetat; $[\alpha]_D^{26}$ = 43,3° (c=1, in Wasser);
2. Fraktion: 239,7 mg Pgl-His-Trp-Ser-Tyr-D-Trp-Ser(Ac-$\beta$-L-Fuc)-Arg-Pro-NH-$C_2H_5$-acetat; $[\alpha]_D^{26}$ = -49,7° (c=1, in Wasser);
3. Fraktion: 97,9 mg Pgl-His-Trp-Ser-Tyr-D-Trp-Ser($\beta$-L-Fuc)-Arg-Pro-NH-$C_2H_5$-acetat; $[\alpha]_D^{26}$ = -43,7° (c=1, in Wasser).

8. Beispiel:

Pgl-His-Trp-Ser-Tyr-D-Trp-Ser($\alpha$-D-Man)-Arg-Pro-NH-$C_2H_5$,
Pgl-His-Trp-Ser-Tyr-D-Trp-Ser(Ac-$\alpha$-D-Man)-Arg-Pro-NH-$C_2H_5$ und
Pgl-His-Trp-Ser-Tyr-D-Trp-Ser($Ac_2$-$\alpha$-D-Man)-Arg-Pro-NH-$C_2H_5$

8a. Fmoc-Ser($Ac_4$-$\alpha$-D-Man)-OBzl

10 g (24 mmol) Fmoc-Ser-OBzl werden in einer Mischung aus 80 ml Toluol und 80 ml Nitromethan gelöst. Nach der Zugabe von 9,8 g (24 mmol) 2,3,4,6-Tetra-O-acetyl-$\alpha$-D-mannopyranosylbromid und 6,1 g (24 mmol) $Hg(CN)_2$ wird der Reaktionsansatz 15 Stunden bei 40°C gerührt. Der Verlauf der Glycosidierung wird dünnschichtchromatographisch verfolgt (Laufmittel: Dichlormethan/Essigsäureethylester wie 6:1; Kieselgel-Fertigfolie-Merck, $GF_{254}$; Detektion: Ethanol/Schwefelsäure wie 10:1 (v/v) und anschließender Wärmebehandlung). Der auf 0°C abgekühlte Reaktionsansatz wird zweimal mit einer 10%igen wäßrigen Kaliumjodid-Lösung, einmal mit gesättigter wäßrigen Natriumhydrogencarbonat-Lösung und einmal mit Eiswasser gewaschen. Die organische Phase wird im Vakuum eingeengt und der verbleibende Sirup wird zweimal mit Toluol codestilliert. Das resultierende Produkt (24 g) wird säulenchromatographisch an Kieselgel gereinigt (Kieselgel 60 (70-230 mesh); Elutionsmittel; Chloroform/Essigsäureethylester wie 9:1).

Ausbeute 14,5 g (80,8 %).

8b. Fmoc-Ser($Ac_4$-$\alpha$-D-Man)-OH

14,5 g Fmoc-Ser($Ac_4$-$\alpha$-D-Man)-OBzl werden in 100 ml trockenem Essigsäureethylester gelöst, mit 14,5 g Palladium/Kohle (10%ig) versetzt und bei Raumtemperatur 70 Minuten hydriert. Nach Abfiltrieren des Katalysators wird die organische Phase einmal mit Eiswasser gewaschen, anschließend über Natriumsulfat getrocknet und im Vakuum zum Sirup eingeengt. Das resultierende Produkt wird säulenchromatographisch an 130 g Kieselgel (Elutionsmittel: Dichlormethan/Aceton 4:1) gereinigt.

Ausbeute 11,8 g (93 %); $[\alpha]_D^{20}$ = +37° (c=1, in Essigsäureethylester).

$^{13}C$-NMR (90 MHz, $CDCl_3$): $\delta$ = 172,97 (COOH); 170,67 (2 x CO, Ac); 170,25 (CO, Ac); 169,70 (CO, Ac); 156,15 (CO, Ureth); 143,75 + 141,15 + 127,66 + 127,61 + 125,06 + 119,91 (Aryl, Fmoc); 98,02 (C-1, Man); 69,41 (CH, Fmoc); 69,41 + 68,87 (2 x C) + 67,30 + 66,05 (C-2, C-3, C-4, C-5, C-6, Man); 62,26 ($CH_2$, Ser); 54,40 (CH, Ser); 47,04 ($CH_2$, Fmoc); 20,70 (2 x C) + 20,59 (2 x C) ($CH_3$, Ac).

8c. H-Ser($Ac_4$-$\alpha$-D-Man)-Arg-Pro-NH-$C_2H_5$y-tosylat

4,6 g (7 mmol) Fmoc-Ser($Ac_4$-α-D-Man)-OH werden analog Beispiel 1d mit 4,5 g H-Arg-Pro-NH-$C_2H_5$-ditosylat umgesetzt.

Der resultierende Schaum wird analog Beispiel 6d umgesetzt.

Ausbeute 6,24 g, amorph; $[\alpha]_D^{22}$ = -9,2° (c=1, in Wasser).

8d. Fmoc-D-Trp-Ser($Ac_4$-α-D-Man)-Arg-Pro-NH-$C_2H_5$-tosylat

2,15 g (5 mmol) Fmoc-D-Trp-OH werden analcg Beispiel 6d mit 4,44 g H-Ser($Ac_4$-α-D-Man)-Arg-Pro-NH-$C_2H_5$-tosylat umgesetzt.

Ausbeute 5,75 g, amorph; $[\alpha]_D^{22}$ = -3,4° (c=1, in Methanol).

8e. H-D-Trp-Ser-($Ac_4$-α-D-Man)-Arg-Pro-NH-$C_2H_5$-tosylat

5,19 g (4 mmol) Fmoc-D-Trp-Ser($Ac_4$-α-D-Man)-Arg-Pro-NH-$C_2H_5$-tosylat werden analog Beispiel 3d und 4c umgesetzt.

Ausbeute 3,05 g, amorph; $[\alpha]_D^{22}$ = - 41,7° (c=1, in Wasser).

8f. Z-Trp-Ser-Tyr-D-Trp-Ser($Ac_4$-α-D-Man)-Arg-Pro-NH-$C_2H_5$-tosylat

1,48 g (2,5 mmol) Z-Trp-Ser-Tyr-OH werden analog Beispiel 3e mit 2,68 g H-D-Trp-Ser($Ac_4$-α-D-Man)-Arg-Pro-NH-$C_2H_5$-tosylat umgesetzt.

Ausbeute 3,55 g, amorph; $[\alpha]_D^{22}$ = -17,0° (c=1, in Methanol).

8g. H-Trp-Ser-Tyr-D-Trp-Ser($Ac_4$-α-D-Man)-Arg-Pro-NH-$C_2H_5$-ditosylat

3,3 g (2 mmol) Z-Trp-Ser-Tyr-D-Trp-Ser($Ac_4$-α-D-Man)-Arg-Pro-NH-$C_2H_5$-tosylat werden analog Beispiel 4e katalytisch hydriert.

Ausbeute 2,85 g, amorph; $[\alpha]_D^{23}$ = -12,6° (c=1, in Methanol).

8h. Pgl-His-Trp-Ser-Tyr-D-Trp-Ser(α-D-Man)-Arg-Pro-NH-$C_2H_5$-acetat, Pgl-His-Trp-Ser-Tyr-D-Trp-Ser(Ac-α-D-Man)-Arg-Pro-NH-$C_2H_5$-acetat und Pgl-His-Trp-Ser-Tyr-D-Trp-Ser($Ac_2$-α-D-Man)-Arg-Pro-NH-$C_2H_5$-acetat

0,45 g (1 mmol) Pgl-His(Dnp)-OH werden analog Beispiel 1g mit 1,68 g H-Trp-Ser-Tyr-D-Trp-Ser($Ac_4$-α-D-Man)-Arg-Pro-NH-$C_2H_5$-ditosylat umgesetzt.

Ausbeute an Rohsubstanz-acetat: 1,43 g.

Nach chromatographischer Reinigung:

1. Fraktion: 109,6 mg Pgl-His-Trp-Ser-Tyr-D-Trp-Ser($Ac_2$-α-D-Man)-Arg-Pro-NH-$C_2H_5$; $[\alpha]_D^{25}$ = -33,4° (c=1, in Wasser);
2. Fraktion: 346,6 mg Pgl-His-Trp-Ser-Tyr-D-Trp-Ser(Ac-α-D-Man)-Arg-Pro-NH-$C_2H_5$; $[\alpha]_D^{25}$ = -33,6° (c=1, in Wasser);
3. Fraktion: 76, 8 mg Pgl-His-Trp-Ser-Tyr-D-Trp-Ser(α-D-Man)-Arg-Pro-NH-$C_2H_5$; $[\alpha]_D^{25}$ = -27,5° (c=1, in Wasser),

Mischfraktion zwischen Fraktion 1 und 2: 187,5 mg und

Mischfraktion zwischen Fraktion 2 und 3: 76,2 mg.

9. Beispiel:

Pgl-His-Trp-Ser-Tyr-D-Trp-Ser(β-D-Xyl)-Arg-Pro-NH-$C_2H_5$ und

Pgl-His-Trp-Ser-Tyr-D-Trp-Ser(Ac-β-D-Xyl)-Arg-Pro-NH-$C_2H_5$

9a. Fmoc-Ser($Ac_3$-β-D-Xyl)-OBzl

Wird analog Beispiel 18a aus Fmoc-Ser-OBzl und 2,3,4-Tri-O-acetyl-α-D-xylopyranosylbromid hergestellt.

9b. Fmoc-Ser($Ac_3$-β-D-Xyl)-OH

Wird analog Beispiel 18b aus Fmoc-Ser($Ac_3$-β-D-Xyl)-OBzl durch katalytische Hydrierung hergestellt.

$[\alpha]_D^{20}$ = -19,9° (c=1, in Essigsäureethylester).

9c. H-Ser($Ac_3$-β-D-Xyl)-Arg-Pro-NH-$C_2H_5$-tosylat

4,1 g (7 mmol) Fmoc-Ser($Ac_3$-β-D-Xyl)-OH werden analog Beispiel 1d mit 4,5 g H-Arg-Pro-NH-$C_2H_5$-ditosylat umgesetzt.
Der resultierende Schaum wird analog Beispiel 6c weiterverarbeitet.
Ausbeute 4,93 g, amorph; $[\alpha]_D^{23}$ = -57,0° (c=1, in Wasser).

9d. Fmoc-D-Trp-Ser($Ac_3$-β-D-Xyl)-Arg-Pro-NH-$C_2H_5$-tosylat

2,15 g (5 mmol) Fmoc-D-Trp-OH werden analog Beispiel 6d mit 4,08 g H-Ser($Ac_3$-β-D-Xyl)-Arg-Pro-NH-$C_2H_5$-tosylat umgesetzt.
Ausbeute 5,7 g, amorph; $[\alpha]_D^{23}$ = -41,5° (c=1, in Methanol).

9e. H-D-Trp-Ser($Ac_3$-β-D-Xyl)-Arg-Pro-NH-$C_2H_5$-tosylat

4,9 g (4 mmol) Fmoc-D-Trp-Ser($Ac_3$-β-D-Xyl)-Arg-Pro-NH-$C_2H_5$-tosylat werden analog Beispiel 3d und 4c umgesetzt.
Ausbeute 2,58 g, amorph; $[\alpha]_D^{23}$ = -93,7° (c=1, in Wasser).

9f. Z-Trp-Ser-Tyr-D-Trp-Ser($Ac_3$-β-D-Xyl)-Arg-Pro-NH-$C_2H_5$-tosylat

1,48 g (2,5 mmol) Z-Trp-Ser-Tyr-OH werden analog Beispiel 3e mit 2,5 g H-D-Trp-Ser($Ac_3$-β-D-Xyl)-Arg-Pro-NH-$C_2H_5$-tosylat umgesetzt.
Ausbeute 3,39 g, amorph; $[\alpha]_D^{23}$ = -48,5° (c=1, in Methanol).

9g. H-Trp-Ser-Tyr-D-Trp-Ser($Ac_3$-β-D-Xyl)-Arg-Pro-NH-$C_2H_5$-ditosylat

2,78 g (2 mmol) Z-Trp-Ser-Tyr-D-Trp-Ser($Ac_3$-β-D-Xyl)-Arg-Pro-NH-$C_2H_5$-tosylat werden analog Beispiel 4e katalytisch hydriert.
Ausbeute 2,5 g, amorph; $[\alpha]_D^{25}$ = -46,1° (c=1, in Methanol).

9h. Pgl-His-Trp-Ser-Tyr-D-Trp-Ser(β-D-Xyl)-Arg-Pro-NH-$C_2H_5$-acetat und Pgl-His-Trp-Ser-Tyr-D-Trp-Ser(Ac-β-D-Xyl)-Arg-Pro-NH-$C_2H_5$

0,45 g Pgl-His(Dnp)-OH werden analog Beispiel 1 g mit 1,61 g H-Trp-Ser-Tyr-D-Trp-Ser($Ac_3$-β-D-Xyl)-Arg-Pro-NH-$C_2H_5$-ditosylat umgesetzt.
Ausbeute an Rohsubstanz-acetat: 0,9344 g.
Ausbeute nach chromatographischer Reinigung:
1. Fraktion: 407,3 mg Pgl-His-Trp-Ser-Tyr-D-Trp-Ser(β-D-Xyl)-Arg-Pro-NH-$C_2H_5$-acetat; $[\alpha]_D^{25}$ = -59,7° (c=1, in Wasser) und
2. Fraktion: 90,3 mg Pgl-His-Trp-Ser-Tyr-D-Trp-Ser(Ac-β-D-Xyl)-Arg-Pro-NH-$C_2H_5$-acetat; $[\alpha]_D^{25}$ = -56,6° (c=1, in Wasser).

10. Beispiel:

Pgl-His-Trp-Ser-Tyr-D-Trp-Ser(α-L-Rha)-Arg-Pro-NH-$C_2H_5$ und
Pgl-His-Trp-Ser-Tyr-D-Trp-Ser(Ac-α-L-Rha)-Arg-Pro-NH-$C_2H_5$

10a. Fmoc-Ser($Ac_3$-α-L-Rha)-OBzl

Analog Beispiel 8a werden Fmoc-Ser-OBzl und 2,3,4-Tri-O-acetyl-α-L-rhamnopyranosylbromid umgesetzt.

10b. Fmoc-Ser($Ac_3$-α-L-Rha)-OH

Fmoc-Ser($Ac_3$-α-L-Rha)-OBzl werden analog Beispiel 8b katalytisch hydriert.

$[\alpha]_D^{20}$ = -28,7° (c = 1, in Essigsäureethylester).

10c. H-Ser($Ac_3$-α-L-Rha)-Arg-Pro-NH-$C_2H_5$-tosylat

4,2 g (7 mmol) Fmoc-D-Ser($Ac_3$-α-L-Rha)-OH werden analog Beispiel 1d mit 4,5 g H-Arg-Pro-NH-$C_2H_5$-ditosylat umgesetzt.
Der resultierende Schaum wird analog Beispiel 6c weiterverarbeitet.
Ausbeute 4,83 g, amorph; $[\alpha]_D^{26}$ = -61,4° (c = 1, in Wasser).

10d. Fmoc-D-Trp-Ser($Ac_3$-α-L-Rha)-Arg-Pro-NH-$C_2H_5$-tosylat

2,14 g (5 mmol) Fmoc-D-Trp-OH werden analog Beispiel 6d mit 4,15 g H-Ser($Ac_3$-α-L-Rha)-Arg-Pro-NH-$C_2H_5$-tosylat umgesetzt.
Ausbeute 5,85 g, amorph; $[\alpha]_D^{26}$ = -38,8° (c = 1, in Methanol).

10e. H-D-Trp-Ser($Ac_3$-α-L-Rha)-Arg-Pro-NH-$C_2H_5$-tosylat

4,95 (4 mmol) Fmoc-D-Trp-Ser($Ac_3$-α-L-Rha)-Arg-Pro-NH-$C_2H_5$-tosylat werden analog Beispiel 3d und 4c umgesetzt.
Ausbeute 2,72 g, amorph; $[\alpha]_D^{26}$ = -95,8° (c = 1, in Wasser).

10f. Z-Trp-Ser-Tyr-D-Trp-Ser($Ac_3$-α-L-Rha)-Arg-Pro-NH-$C_2H_5$-tosylat

1,48 g (2,5 mmol) Z-Trp-Ser-Tyr-OH werden analog Beispiel 3e mit 2,54 g H-D-Trp-Ser($Ac_3$-α-L-Rha)-Arg-Pro-NH-$C_2H_5$-tosylat umgesetzt.
Ausbeute 3,95 g, amorph; $[\alpha]_D^{26}$ = -44,4° (c = 1, in Methanol).

10g. H-Trp-Ser-Tyr-D-Trp-Ser($Ac_3$-α-L-Rha)-Arg-Pro-NH-$C_2H_5$-ditosylat

3,2 g (2 mmol) Z-Trp-Ser-Tyr-D-Trp-Ser($Ac_3$-α-L-Rha)-Arg-Pro-NH-$C_2H_5$-tosylat werden analog Beispiel 4e katalytisch hydriert.
Ausbeute 2,87 g, amorph; $[\alpha]_D^{26}$ = -42,3° (c = 1, in Methanol).

10h. Pgl-His-Trp-Ser-Tyr-D-Trp-Ser(α-L-Rha)-Arg-Pro-NH-$C_2H_5$-acetat und Pgl-His-Trp-Ser-Tyr-D-Trp-Ser-(Ac-α-L-Rha)-Arg-Pro-NH-$C_2H_5$-acetat

0,45 g (1 mmol) Pgl-His(Dnp)-OH werden analog Beispiel 1g mit 1,62 g H-Trp-Ser-Tyr-D-Trp-Ser($Ac_3$-α-L-Rha)-Arg-Pro-NH-$C_2H_5$-ditosylat umgesetzt.
Ausbeute an Rohsubstanz-acetat: 893 mg.
Ausbeute nach chromatographischer Reinigung:
1. Fraktion: 524,3 mg Pgl-His-Trp-Ser-Tyr-D-Trp-Ser(α-L-Rha)-Arg-Pro-NH-$C_2H_5$-acetat;
2. Fraktion: 95,6 mg Pgl-His-Trp-Ser-Tyr-D-Trp-Ser(Ac-α-L-Rha)-Arg-Pro-NH-$C_2H_5$-acetat und
Mischfraktion: 87,1 mg.

11. Beispiel:

Pgl-His-Trp-Ser-Tyr-D-Ser(β-D-Gal)-Leu-Arg-Pro-NH-$C_2H_5$

11a. Fmoc-D-Ser($Ac_4$-β-D-Gal)-OBzl

Analog Beispiel 8a werden Fmoc-D-Ser-OBzl und 2,3,4,6-Tetra-O-acetyl-β-D-galacto-pyranosylbromid umgesetzt.
$[\alpha]_D^{25}$ = -5,9° (c = 1, in Chloroform).

11b. Fmoc-D-Ser($Ac_4$-β-D-Gal)-OH

Fmoc-D-Ser($Ac_4$-β-D-Gal)-OBzl werden analog Beispiel 8b katalytisch hydriert.
$[\alpha]_D^{25}$ = -5,8° (c = 1, in Chloroform).

11c. H-D-Ser($Ac_4$-β-D-Gal)-Leu-Arg-Pro-NH-$C_2H_5$-tosylat

4,6 g (7 mmol) Fmoc-D-Ser($Ac_4$-β-D-Gal)-OH werden analog Beispiel 1d mit 5,3 g H-Leu-Arg-Pro-NH-$C_2H_5$-ditosylat umgesetzt.
Der schaumige Rückstand wird analog Beispiel 3d und 4e umgesetzt und gereinigt.
Ausbeute 5,38 g, amorph; $[\alpha]_D^{26}$ = -54,5° (c=1, in Wasser).

11d. H-Trp-Ser-Tyr-OH-acetat

23,55 g (40 mmol) Z-Trp-Ser-Tyr-OH werden in 700 ml 90 %iger wäßriger Essigsäure gelöst und mit Pd/Kohle katalytisch hydriert. Nach beendigter Hydrierung wird der Katalysator abgesaugt und das Filtrat eingeengt. Der Rückstand wird mit Ether verrieben, abgesaugt und getrocknet.
Ausbeute 20,4 g; Schmp. 176-181° unter Zersetzung; $[\alpha]_D^{25}$ = +33,5° (c=1, in Methanol).

11e. Fmoc-Trp-Ser-Tyr-OH

Zu einer Mischung aus 50 ml Wasser und 50 ml Dioxan gibt man nacheinander 19,96 g (38,8 mmol) H-Trp-Ser-Tyr-OH-acetat, 6,52g (77,6 mmol) $NaHCO_3$ und 14,2 g (42 mmol) Fmoc-ONSu. Man läßt 4 Stunden bei Raumtemperatur stehen. Unlösliches wird anderntags abgesaugt und verworfen. Das Filtrat wird eingeengt. Der Rückstand wird mit Petrolether verrieben abgesaugt und getrocknet.
Ausbeute 27,1 g.
Zur Reinigung wird die Substanz mit 400 ml Essigester aufgekocht und nach dem Abkühlen abgesaugt.
Ausbeute 23,1 g (88 %); Schmp. 186-188° C; $[\alpha]_D^{25}$ = -0,4° (c=1, in Methanol).

11f. Fmoc-Trp-Ser-Tyr-D-Ser($Ac_4$-β-D-Gal)-Leu-Arg-Pro-NH-$C_2H_5$-tosylat

3.0 g (3 mmol) H-D-Ser($Ac_4$-β-D-Gal)-Leu-Arg-Pro-NH-$C_2H_5$-tosylat werden analog Beispiel 3e mit 2,03 g Fmoc-Trp-Ser-Tyr-OH, 0,49 g HOOBt und 0,66 g DCC in 10 ml Dimethylformamid umgesetzt. Der Rückstand wird zwischen n-Pentanol und Wasser verteilt. Die n-Pentanol-Phase wird eingeengt und der Rückstand mit Methyl-tert.-butylether verrieben. Der Niederschlag wird abgesaugt und getrocknet.
Ausbeute 4,69 g; Schmp. 108-110° C unter Zersetzung; $[\alpha]_D^{23}$ = -34,2° (c=1, in Methanol).

11g. H-Trp-Ser-Tyr-D-Ser($Ac_4$-β-D-Gal)-Leu-Arg-Pro-NH-$C_2H_5$-tosylat

Zu einer Lösung von 2,98 g (2 mmol) Fmoc-Trp-Ser-Tyr-D-Ser($Ac_4$-β-D-Gal)-Leu-Arg-Pro-NH-$C_2H_5$-tosylat in 15 ml Dimethylformamid gibt man bei Raumtemperatur 0,2 ml (2 mmol) Diethylamin. Man rührt 1 Stunde bei Raumtemperatur und engt ein. Der Rückstand wird mit Diethylether verrieben, abgesaugt und getrocknet.
Ausbeute 2,65 g; Schmp. 104-105° C unter Zersetzung; $[\alpha]_D^{23}$ = -34,5° C (c=1, in Methanol).

11h. Pgl-His-Trp-Ser-Tyr-D-Ser(β-D-Gal)-Leu-Arg-Pro-NH$C_2H_5$-acetat

Zu einer Lösung von 1,27 g (1 mmol) H-Trp-Ser-Tyr-D-Ser($Ac_4$-β-D-Gal)-Leu-Arg-Pro-NH-$C_2H_5$-tosylat, 163 mg HOOBt und 450 g Pgl-His(Dnp)-OH in 6 ml Dimethylacetamid gibt man 225 mg DCC und läßt 1 Stunde bei 0° C und 3 Stunden bei Raumtemperatur rühren, läßt über Nacht stehen und gibt anderntags 1 ml Hydrazinhydrat zu. Man läßt 4 Stunden bei Raumtemperatur rühren, saugt den Niederschlag ab und versetzt das Filtrat mit Essigester. Der Niederschlag wird abgesaugt und aus Methanol/Essigester umgefällt, abgesaugt mit Essigester gewaschen und getrocknet.
Ausbeute 910 mg.
Analog Beispiel 1g wird die Substanz in das Acetat überführt: Ausbeute 818 mg.
Ausbeute nach chromatographischer Reinigung: 413 mg; $[\alpha]_D^{22}$ = -40,8° (c=1, in Wasser).

12. Beispiel:

Pgl-His-Trp-Ser-Tyr-D-Ser(L-Ara)-Leu-Arg-Pro-NH-$C_2H_5$

12a. Fmoc-D-Ser($Ac_3$-L-Ara)-OBzl

Analog Beispiel 8a weden Fmoc-D-Ser-OBzl und 2,3,4-Tri-O-acetyl-L-arabinopyranosylbromid umgesetzt.
$[\alpha]_D^{25}$ = -9,3° (c=1, in Chloroform).

12b. Fmoc-D-Ser($Ac_3$-L-Ara)-OH

Fmoc-D-Ser($Ac_3$-L-Ara)-OBzl werden analog Beispiel 8b katalytisch hydriert.
$[\alpha]_D^{25}$ = -21,9° (c=1, in Chloroform)

12c. H-D-Ser($Ac_3$-L-Ara)-Leu-Arg-Pro-NH-$C_2H_5$-tosylat

4,1 g (7 mmol) Fmoc-D-Ser($Ac_3$-L-Ara)-OH werden analog Beispiel 1d mit 5,3 g H-Leu-Arg-Pro-NH-$C_2H_5$-ditosylat umgesetzt.
Der schaumige Rückstand wird analog Beispiel 3d und 4e umgesetzt und gereinigt.
Ausbeute 5,1 g; $[\alpha]_D^{23}$ = -57,5° (c=1, in Wasser).

12d. Fmoc-Trp-Ser-Tyr-D-Ser($A_3$-L-Ara)-Leu-Arg-Pro-NH-$C_2H_5$-tosylat

2,78 g (3 mmol) H-D-Ser($Ac_3$-L-Ara)-Leu-Arg-Pro-NH-$C_2H_5$-tosylat werden analog Beispiel 11f mit 2,03 g Fmoc-Trp-Ser-Tyr-OH umgesetzt. Der Niederschlag wird anderntags abgesaugt und das Filtrat mit 150 ml Wasser, 3 ml gesättigter $NaHCO_3$-Lösung und einigen ml gesättigter NaCl-Lösung versetzt. Dabei fällt ein Niederschlag aus, der abgesaugt wird.
Ausbeute 3,47 g; Schmp. 128-131 °C unter Zersetzung; $[\alpha]_D^{23}$ = -36,3° (c=1, in Methanol).

12e. H-Trp-Ser-Tyr-D-Ser($Ac_3$-L-Ara)-Leu-Arg-Pro-NH-$C_2H_5$-tosylat

2,83 g (2 mmol) Fmoc-Trp-Ser-D-Ser($Ac_3$-L-Ara)-Leu-Arg-Pro-NH-$C_2H_5$-tosylat werden analog Beispiel 11g umgesetzt.
Ausbeute 2,55 g; Schmp. 106-108 °C unter Zersetzung; $[\alpha]_D^{23}$ = -37,6° (c=1, in Methanol).

12f. Pgl-His-Trp-Ser-Tyr-D-Ser(L-Ara)-Leu-Arg-Pro-NH-$C_2H_5$-acetat

1,19 g (1 mmol) H-Trp-Ser-Tyr-D-Ser($Ac_3$-L-Ara)-Leu-Arg-Pro-NH-$C_2H_5$-tosylat werden analog Beispiel 11h umgesetzt.
Ausbeute an Roh-Acetat: 1,05 g.
Ausbeute nach chromatographischer Reinigung 362 mg; $[\alpha]_D^{22}$ = -46,0° (c=1, in Wasser).

13. Beispiel:

Pgl-His-Trp-Ser-Tyr-D-Ser(β-Lac)-Leu-Arg-Pro-NH-$C_2H_5$

13a. Fmoc-D-Ser($Ac_7$-β-Lac)-OBzl

Analog Beispiel 8a werden Fmoc-D-Ser-OBzl und 2,3,6,2′,3′,4′,6′-Hepta-O-Acetyl-β-lactopyranosylbromid umgesetzt.
$[\alpha]_D^{22}$ = -9,1° (c=1, in Chloroform).

13b. Fmoc-D-Ser($Ac_7$-β-Lac)-OH

Fmoc-D-Ser($Ac_7$-β-Lac) werden analog Beispiel 8b katalytisch hydriert.
$[\alpha]_D^{22}$ = -23,7° (c=1, in Chloroform).

13c. H-D-Ser($Ac_7$-β-Lac)-Leu-Arg-Pro-NH-$C_2H_5$-tosylat

4,5 g (4,75 mmol) Fmoc-D-Ser($Ac_7$-β-Lac)-OH werden analog Beispiel 1d mit 3,6 g (4,75 mmol) H-Leu-Arg-Pro-NH-$C_2H_5$-ditosylat umgesetzt.
Der schaumige Rückstand wird analog Beispiel 3d und 4e umgesetzt und gereinigt.
Ausbeute 4,32 g; $[\alpha]_D^{21}$ = -49,5° (c=1, in Wasser).

13d. Fmoc-Trp-Ser-Tyr-D-Ser($Ac_7$-β-Lac)-Leu-Arg-Pro-NH-$C_2H_5$-tosylat

3,87 g (3 mmol) H-D-Ser($Ac_7$-β-Lac)-Leu-Arg-Pro-NH-$C_2H_5$-tosylat werden analog Beispiel 11f mit 2,03 g Fmoc-Trp-Ser-Tyr-OH umgesetzt.
Ausbeute 5,56 g; Schmp. 124-127 °C unter Zersetzung; $[\alpha]_D^{21}$ = -34,5° (c=1, in Methanol).

13e. H-Trp-Ser-Tyr-D-Ser($Ac_7$-β-Lac)-Leu-Arg-Pro-NH-$C_2H_5$-tosylat

3,9 g (2 mmol) Fmoc-Trp-Ser-Tyr-D-Ser($Ac_7$-β-Lac)-Leu-Arg-Pro-NH-$C_2H_5$-tosylat werden analog Beispiel 11g umgesetzt.
Ausbeute 3,36 g; Schmp. 84-90 °C unter Zersetzung; $[\alpha]_D^{21}$ = -35,3° (c=1, in Methanol).

13f. Pgl-His-Trp-Ser-Tyr-D-Ser(β-Lac)-Leu-Arg-Pro-NH-$C_2H_5$-acetat

1,72 g (1 mmol) H-Trp-Ser-Tyr-D-Ser($Ac_7$-β-Lac)-Leu-Arg-Pro-NH-$C_2H_5$-tosylat werden analog Beispiel 11h umgesetzt.
Ausbeute an Rohacetat: 1,25 g.
Ausbeute nach chromatographischer Reinigung: 608 mg, $[\alpha]_D^{22}$ = -33,4° (c=1, in Wasser).

14. Beispiel:

Pgl-His-Trp-Ser-Tyr-D-Ser(β-L-Xyl)-Leu-Arg-Pro-NH-$C_2H_5$

14a. Fmoc-D-Ser(β-L-Xyl)-OBzl

Analog Beispiel 8a werden Fmoc-D-Ser-OBzl und 2,3,4-Tri-O-acetyl-β-L-xylo-pyranosylbromid umgesetzt.

14b. Fmoc-D-Ser(β-L-Xyl)-OH

Fmoc-D-Ser(β-L-Xyl)-OBzl werden analog Beispiel 8b katalytisch hydriert.
$[\alpha]_D^{22}$ = +20,4° (c=1, in Essigester).

14c. H-D-Ser(β-L-Xyl)-Leu-Arg-Pro-NH-$C_2H_5$-tosylat

4,1 g (7 mmol) Fmoc-D-Ser($Ac_3$-β-L-Xyl)-OH werden analog Beispiel 1d mit 5,3 g H-Leu-Arg-Pro-NH-$C_2H_5$-ditosylat umgesetzt.
Der schaumige Rückstand wird analog Beispiel 3d und 4e umgesetzt und gereinigt.
Ausbeute 4,95 g; $[\alpha]_D^{21}$ = -33,1° (c=1, in Wasser).

14d. Fmoc-Trp-Ser-Tyr-D-Ser($Ac_3$-β-L-Xyl)-Leu-Arg-Pro-NH-$C_2H_5$-tosylat

2,8 g (3 mmol) H-D-Ser($Ac_3$-β-L-Xyl)-Leu-Arg-Pro-NH-$C_2H_5$ werden analog Beispiel 11f mit 2,03 g Fmoc-Trp-Ser-Tyr umgesetzt.
Ausbeute 4,65 g; Schmp. 109-114 °C unter Zersetzung; $[\alpha]_D^{21}$ = -18,7° (c=1, in Methanol).

14e. H-Trp-Ser-Tyr-D-Ser($Ac_3$-β-L-Xyl)-Leu-Arg-Pro-NH-$C_2H_5$-tosylat

3,18 g Fmoc-Trp-Ser-Tyr-D-Ser($Ac_3$-β-L-Xyl)-Leu-Arg-Pro-NH-$C_2H_5$-tosylat werden analog Beispiel 11g umgesetzt.
Ausbeute 2,75 g; Schmp. 98-103 °C unter Zersetzung; $[\alpha]_D^{21}$ = -20,5° (c=1, in Methanol).

14f. Pgl-His-Trp-Ser-Tyr-D-Ser(β-L-Xyl)-Leu-Arg-Pro-NH-$C_2H_5$-acetat

1,36 g H-Trp-Ser-Tyr-D-Ser($Ac_3$-β-L-Xyl)-Leu-Arg-Pro-NH-$C_2H_5$-tosylat (1 mmol) werden analog Beispiel 11h umgesetzt.
Ausbeute an Rohacetat: 988 mg.
Ausbeute nach chromatographischer Reinigung: 278 mg; $[\alpha]_D^{22}$ = -28,5° (c=1, in Wasser).

15. Beispiel:

Pgl-His-Trp-Ser-Tyr-D-Trp-Ser(L-Ara)-Arg-Pro-NH-$C_2H_5$

15a. Fmoc-Ser($Ac_3$-L-Ara)-OBzl

Analog Beispiel 8a werden Fmoc-D-Ser-OBzl und 2,3,4-Tri-0-acetyl-L-arabinopyranosylbromid umgesetzt.
$[\alpha]_D^{22}$ = ±0° (c = 1, in Chloroform).

15b. Fmoc-Ser($Ac_3$-L-Ara)-OH

Fmoc-Ser($Ac_3$-L-Ara)-OBzl werden analog Beispiel 8b katalytisch hydriert.
$[\alpha]_D^{22}$ = +9,8° (c = 1, in Chloroform).

15c. H-Ser($Ac_3$-L-Ara)-Arg-Pro-NH-$C_2H_5$-tosylat

4,1 g (7 mmol) Fmoc-Ser($Ac_3$-L-Ara)-OH werden analog Beispiel 1d mit 4,5 H-Arg-Pro-NH-$C_2H_5$-ditosylat umgesetzt.
Der schaumige Rückstand wird analog Beispiel 6c umgesetzt.
Ausbeute 4,8 g; $[\alpha]_D^{21}$ = -41,3° (c = 1, in Wasser).

15d. Fmoc-D-Trp-Ser($Ac_3$-L-Ara)-Arg-Pro-NH-$C_2H_5$-tosylat

4,08 g (5 mmol) H-Ser($Ac_3$-L-Ara)-Arg-Pro-NH-$C_2H_5$-tosylat werden analog Beispiel 6d mit 2,14 g Fmoc-D-Trp-OH umgesetzt.
Ausbeute 5,7 g; Schmp. 95-97° C unter Zersetzung; $[\alpha]_D^{23}$ = -26,3° (c = 1, in Methanol).

15e. H-D-Trp-Ser($Ac_3$-L-Ara)-Arg-Pro-NH-$C_2H_5$-tosylat

4,88 g (4 mmol) Fmoc-D-Trp-Ser($Ac_3$-L-Ara)-Arg-Pro-NH-$C_2H_5$ werden analog Beispiel 3d und 4c umgesetzt.
Ausbeute 2,23 g; $[\alpha]_D^{23}$ = -71,8° (c = 1, in Wasser).

15f. Fmoc-Trp-Ser-Tyr-D-Trp-Ser($Ac_3$-L-Ara)-Arg-Pro-NH-$C_2H_5$-tosylat

2,5 g (2,5 mmol) H-D-Trp-Ser($Ac_3$-L-Ara)-Arg-Pro-NH-$C_2H_5$-tosylat werden analog Beispiel 11f mit 1,7 g Fmoc-Trp-Ser-Tyr-OH umgesetzt.
Ausbeute 3,65 g; Schmp. 147-149° unter Zersetzung; $[\alpha]_D^{23}$ = -37,3° (c = 1, in Methanol).

15g. H-Trp-Ser-Tyr-D-Trp-Ser($Ac_3$-L-Ara)-Arg-Pro-NH-$C_2H_5$-tosylat

3,3 g (2 mmol) Fmoc-Trp-Ser-Tyr-D-Trp-Ser($Ac_3$-L-Ara)-Arg-Pro-NH-$C_2H_5$ werden analog Beispiel 11g umgesetzt.
Ausbeute 2,3 g; Schmp. 92-94° C unter Zersetzung; $[\alpha]_D^{23}$ = -36,6° (c = 1, in Methanol).

15h. Pgl-His-Trp-Ser-Tyr-D-Trp-Ser(L-Ara)-Arg-Pro-NH-$C_2H_5$-acetat

1,4 g (1 mmol) H-Trp-Ser-Tyr-D-Trp-Ser($Ac_3$-L-Ara)-Arg-Pro-NH-$C_2H_5$-tosylat werden analog Beispiel 11h mit 450 mg Pgl-His(Dnp)-OH umgesetzt.
Ausbeute an Rohacetat: 795,2 mg.
Ausbeute nach chromatographischer Reinigung: 405 mg; $[\alpha]_D^{22}$ = -39,3° (c = 1, in Wasser).

16. Beispiel:

Pgl-His-Trp-Ser-Tyr-D-Trp-Ser(β-L-Xyl)-Arg-Pro-NH-$C_2H_5$

16a. Fmoc-Ser($Ac_3$-β-L-Xyl)-OBzl

Analog Beispiel 8a werden Fmoc-Ser-OBzl und 2,3,4-Tri-O-acetyl-$\beta$-L-xylopyranosyl-bromid umgesetzt.

16b. Fmoc-Ser($Ac_3$-$\beta$-L-Xyl)-OH

Fmoc-Ser($Ac_3$-$\beta$-L-Xyl)-OBzl werden analog Beispiel 8b katalytisch hydriert.
$[\alpha]_D^{22}$ = +54,4° (c=1, in Methanol).

16c. H-Ser($Ac_3$-$\beta$-L-Xyl)-Arg-Pro-NH-$C_2H_5$-tosylat

4,1 g (7 mmol) Fmoc-Ser($Ac_3$-$\beta$-L-Xyl)-OH werden analog Beispiel 1d mit 4,5 g H-Arg-Pro-NH-$C_2H_5$-ditosylat umgesetzt.
Der schaumige Rückstand wird analog Beispiel 6c umgesetzt.
Ausbeute 5,1 g; $[\alpha]_D^{21}$ = -9,6° (c=1, in Wasser).

16d. Fmoc-D-Trp-Ser($Ac_3$-$\beta$-L-Xyl)-Arg-Pro-NH-$C_2H_5$-tosylat

4,08 g (5 mmol) H-Ser($Ac_3$-$\beta$-L-Xyl)-Arg-Pro-NH-$C_2H_5$-tosylat werden analog Beispiel 6d mit 2,15 g Fmoc-D-Trp-OH umgesetzt.
Ausbeute 5,7 g; Schmp. 81-83° C unter Zersetzung; $[\alpha]_D^{21}$ = +1,3° (c=1, in Methanol).

16e. H-D-Trp-Ser($Ac_3$-$\beta$-L-Xyl)-Arg-Pro-NH-$C_2H_5$-tosylat

4,9 g (4 mmol) Fmoc-D-Trp-Ser($Ac_3$-$\beta$-L-Xyl)-Arg-Pro-NH-$C_2H_5$ werden analog Beispiel 3d und 4c umgesetzt. Zur Reinigung wird zwischen Essigester ausgeschüttelt und gefriergetrocknet.
Ausbeute 2,6 g; $[\alpha]_D^{21}$ = -42,5° (c=1, in Wasser).

16f. Fmoc-Trp-Ser-Tyr-D-Trp-Ser($Ac_3$-$\beta$-L-Xyl)-Arg-Pro-NH-$C_2H_5$-tosylat

2,4 g (2,4 mmol) H-D-Ser($Ac_3$-$\beta$-L-Xyl)-Arg-Pro-NH-$C_2H_5$-tosylat werden analog Beispiel 11f mit 1,63 g Fmoc-Trp-Ser-Tyr-OH umgesetzt.
Ausbeute 3,95 g; Schmp. 132-137° unter Zersetzung; $[\alpha]_D^{21}$ = -28,9° (c=1, in Methanol).

16g. H-Trp-Ser-Tyr-D-Trp-Ser($Ac_3$-$\beta$-L-Xyl)-Arg-Pro-NH-$C_2H_5$-tosylat

3,3 g (2 mmol) Fmoc-Trp-Ser-Tyr-D-Trp-Ser($Ac_3$-$\beta$-L-Xyl)-Arg-Pro-NH-$C_2H_5$-tosylat werden analog Beispiel 11g umgesetzt.
Ausbeute 2,74 g; Schmp. 131-140° C unter Zersetzung; $[\alpha]_D^{21}$ = -26,8° (c=1, in Methanol).

16h. Pgl-His-Trp-Ser-Tyr-D-Trp-Ser($\beta$-L-Xyl)-Arg-Pro-NH-$C_2H_5$-acetat

1,44 g (1 mmol) H-Trp-Ser-Tyr-D-Trp-Ser($Ac_3$-$\beta$-L-Xyl)-Arg-Pro-NH-$C_2H_5$-tosylat werden analog Beispiel 11h mit 450 mg Pgl-His(Dnp)-OH umgesetzt.
Ausbeute an Rohacetat: 1,14 g.
Ausbeute nach chromatographischer Reinigung: 533 mg; $[\alpha]_D^{22}$ = -42,3° (c=1, in Wasser).

17. Beispiel:

Pgl-His-Trp-Ser-Tyr-D-Trp-Ser($\beta$-Lac)-Arg-Pro-NH-$C_2H_5$

17a. Fmoc-Ser($Ac_7$-$\beta$-Lac)-OBzl

Analog Beispiel 8a werden Fmoc-Ser-OBzl und 2,3,6,2',3'4',6'-Hepta-O-acetyl-lactopyranosyl-bromid umgesetzt.
$[\alpha]_D^{22}$ = -0,3° (c=1, in Chloroform).

17b. Fmoc-Ser($Ac_7$-$\beta$-Lac)-OH

Fmoc-Ser($Ac_3$-$\beta$-Lac) werden analog Beispiel 8b katalytisch hydriert.

$[\alpha]_D^{22}$ = +10,6° (c=1, in Chloroform).

17c. H-Ser($Ac_7$-β-Lac)-Arg-Pro-NH-$C_2H_5$-tosylat

4,49 g (4,75 mmol) Fmoc-Ser($Ac_7$-β-Lac)-OH werden analog Beispiel 1d mit 3,05 g H-Arg-Pro-NH-$C_2H_5$-ditosylat umgesetzt.
Der schaumige Rückstand wird analog Beispiel 6c umgesetzt.
Ausbeute 4,57 g; $[\alpha]_D^{21}$ = -24,8° (c=1, in Wasser).

17d. Fmoc-D-Trp-Ser($Ac_7$-β-Lac)-Arg-Pro-NH-$C_2H_5$-tosylat

3,53 g (3 mmol) H-Ser($Ac_7$-β-Lac)-Arg-Pro-NH-$C_2H_5$-tosylat werden analog Beispiel 6d mit 1,28 g Fmoc-D-Trp-OH umgesetzt.
Ausbeute 4,4 g; Schmp. 106-109° C unter Zersetzung; $[\alpha]_D^{21}$ = -25,1° (c=1, in Methanol).

17e. H-D-Trp-Ser($Ac_7$-β-Lac)-Arg-Pro-NH-$C_2H_5$-tosylat

3,96 g (2,5 mmol) Fmoc-D-Trp-Ser($Ac_7$-β-Lac)-Arg-Pro-NH-$C_2H_5$-tosylat werden analog Beispiel 3d und 4c umgesetzt.
Ausbeute 2,37 g; $[\alpha]_D^{21}$ = -54,6° (c=1, in Wasser).

17f. Fmoc-Trp-Ser-Tyr-D-Trp-Ser($Ac_7$-β-Lac)-Arg-Pro-NH-$C_2H_5$-tosylat

2,25 g (1,65 mmol) H-D-Trp-Ser($Ac_7$-β-Lac)-Arg-Pro-NH-$C_2H_5$-tosylat weden analog Beispiel 11f mit 1,12 g Fmoc-Trp-Ser-Tyr-OH umgesetzt.
Ausbeute 2,65; Schmp. 109-111° unter Zersetzung; $[\alpha]_D^{21}$ = -30,0° (c=1, in Methanol).

17g. H-Trp-Ser-Tyr-D-Trp-Ser($Ac_7$-β-Lac)-Arg-Pro-NH-$C_2H_5$-tosylat

2,43 g (1,2 mmol) Fmoc-Trp-Ser-Tyr-D-Trp-Ser($Ac_7$-β-Lac)-Arg-Pro-NH-$C_2H_5$-tosylat werden analog Beispiel 11g umgesetzt.
Ausbeute 1,93 g; Schmp. 136-142° C unter Zersetzung; $[\alpha]_D^{21}$ = -33,3° (c=1, in Methanol).

17h. Pgl-His-Trp-Ser-Tyr-D-Trp-Ser(β-Lac)-Arg-Pro-NH-$C_2H_5$-acetat

1,8 g (1 mmol) H-Trp-Ser-Tyr-D-Trp-Ser($Ac_7$-β-Lac)-Arg-Pro-NH-$C_2H_5$-tosylat werden analog Beispiel 11h mit 450 mg Pgl-His(Dnp)-OH umgesetzt.
Ausbeute an Rohacetat: 1,24 g.
Ausbeute nach chromatographischer Reinigung: 579 mg; $[\alpha]_D^{22}$ = -39,6° (c=1, in Wasser).

18. Beispiel:

Pgl-His-Trp-Ser-Tyr-D-Trp-Ser(β-D-Glc-NAc)-Arg-Pro-NH-$C_2H_5$

18a. Fmoc-Ser($Ac_3$-β-D-Glc-NAc)-OBzl

Analog Beispiel 8a werden Fmoc-Ser-OBzl und 2-N-Acetyl-3,4,6-Tri-O-acetyl-glucosaminyl-bromid umgesetzt.
$[\alpha]_D^{22}$ = -6,6° (c=1, in Chloroform/Methanol wie 3:1).

18b. Fmoc-Ser($Ac_3$-β-D-Glc-NAc)-OH

Fmoc-Ser($Ac_3$-β-D-Glc-NAc)-OBzl werden analog Beispiel 8b katalytisch hydriert.
$[\alpha]_D^{22}$ = 1,6° (c=1, in Chloroform/Methanol wie 3:1).

18c. H-Ser($Ac_3$-β-D-Glc-NAc)-Arg-Pro-NH-$C_2H_5$-tosylat

3,37 g (5 mmol) Fmoc-Ser($Ac_3$-β-D-Glc-NAc)-OH werden analog Beispiel 1d mit 3,21 g H-Arg-Pro-NH-

$C_2H_5$-ditosylat umgesetzt.

Der schaumige Rückstand wird analog Beispiel 6c umgesetzt.
Ausbeute 4,21 g; $[\alpha]_D^{22}$ = -47,8° (c=1, in Wasser).

18d. Fmoc-D-Trp-Ser($Ac_3$-β-D-Glc-NAc)-Arg-Pro-NH-$C_2H_5$-tosylat

3,61 g (4 mmol) H-Ser($Ac_3$-β-D-Glc-NAc)-Arg-Pro-NH-$C_2H_5$-tosylat werden analog Beispiel 6d mit 1,71 g Fmoc-D-Trp-OH umgesetzt.

Ausbeute 4,95 g; Schmp. 93-95° C unter Zersetzung; $[\alpha]_D^{22}$ = -32,3° (c=1, in Methanol).

18e. H-D-Trp-Ser($Ac_3$-β-D-Glc-NAc)-Arg-Pro-NH-$C_2H_5$-tosylat

4,49 g (3,5 mmol) Fmoc-D-Trp-Ser($Ac_3$-β-D-Glc-NAc)-Arg-Pro-NH-$C_2H_5$-tosylat werden analog Beispiel 3d und 4c umgesetzt.

Ausbeute 3,68 g; Schmp. 105-107° C unter Zersetzung.

18f. Fmoc-Trp-Ser-Tyr-D-Trp-Ser($Ac_3$-β-D-Glc-NAc)-Arg-Pro-NH-$C_2H_5$-tosylat

3,27 g (3 mmol) H-D-Trp-Ser($Ac_3$-β-D-Glc-NAc)-Arg-Pro-NH-$C_2H_5$-tosylat werden analog Beispiel 11f mit 2,03 g Fmoc-Trp-Ser-Tyr-OH umgesetzt.

Ausbeute 4,24 g; Schmp. 145-146° C unter Zersetzung; $[\alpha]_D^{22}$ = -17,6° (c=1, in Methanol).

18g. H-Trp-Ser-Tyr-D-Trp-Ser($Ac_3$-β-D-Glc-NAc)-Arg-Pro-NH-$C_2H_5$-tosylat

3,5 g (2 mmol) Fmoc-Trp-Ser-Tyr-D-Trp-Ser($Ac_3$-β-D-Glc-NAc)-Arg-Pro-NH-$C_2H_5$-tosylat werden analog Beispiel 11g umgesetzt.

Ausbeute 3,05 g; Schmp. 104-107° C unter Zersetzung; $[\alpha]_D^{22}$ = -16,3° (c=1, in Methanol).

18h. Pgl-His-Trp-Ser-Tyr-D-Trp-Ser($Ac_3$-β-Glc-NAc)-Arg-Pro-NH-$C_2H_5$-tosylat

1,52 g (1 mmol) H-Trp-Ser-Tyr-D-Trp-Ser($Ac_3$-β-D-Glc-NAc)-Arg-Pro-NH-$C_2H_5$ werden analog Beispiel 11h mit 450 g Pgl-His(Dnp)-OH umgesetzt.

Ausbeute an Rohacetat: 1,178 g.
Ausbeute nach chromatographischer Reinigung: 353 mg; $[\alpha]_D^{22}$ = -50,8° (c=1, in Wasser).

19. Beispiel:

Pgl-His-Trp-Ser-Tyr-D-Trp-Ser(D-Rib)-Arg-Pro-NH-$C_2H_5$

19a. Fmoc-Ser($Ac_3$-D-Rib)-OBzl

Analog Beispiel 8a werden Fmoc-Ser-OBzl und 2,3,4-Tri-O-acetyl-D-ribopyranosylbromid umgesetzt.
$[\alpha]_D^{22}$ = -51,3° (c=1, in Chloroform).

19b. Fmoc-Ser($Ac_3$-D-Rib)-OBzl

Fmoc-Ser($Ac_3$-D-Rib)-OBzl werden analog Beispiel 8b katalytisch hydriert.
$[\alpha]_D^{22}$ = -23,8° (c=1, in Chloroform).

19c. H-Ser($Ac_3$-D-Rib)-Arg-Pro-NH-$C_2H_5$-tosylat

4,1 g (7 mmol) Fmoc-Ser($Ac_3$-D-Rib)-OH werden analog Beispiel 1d mit 4,5 g H-Arg-Pro-NH-$C_2H_5$-ditosylatumgesetzt.

Der schaumige Rückstand wird analog Beispiel 6c umgesetzt.
Ausbeute 5,45 g; $[\alpha]_D^{22}$ = -65,7° (c=1, in Wasser).

19d. Fmoc-D-Trp-Ser($Ac_3$-D-Rib)-Arg-Pro-NH-$C_2H_5$-tosylat

4,08 g (5 mmol) H-Ser($Ac_3$-D-Rib)-Arg-Pro-NH-$C_2H_5$-tosylat werden analog Beispiel 6d mit 2,15 g Fmoc-D-Trp-OH umgesetzt.

Ausbeute 5,74 g; Schmp. 104-107 °C unter Zersetzung; $[\alpha]_D^{22}$ = -40,7° (c=1, in Methanol).

19e. H-D-Trp-Ser($Ac_3$-D-Rib)-Arg-Pro-NH-$C_2H_5$-tosylat

4,21 g (4 mmol) Fmoc-D-Trp-Ser($Ac_3$-D-Rib)-Arg-Pro-NH-$C_2H_5$-tosylat werden analog Beispiel 3d und 4c umgesetzt.

Ausbeute 3,22 g; Schmp. 98-101 °C; $[\alpha]_D^{22}$ = -27,0° (c=1, in Methanol).

19f. Fmoc-Trp-Ser-Tyr-D-Trp-Ser($Ac_3$-D-Rib)-Arg-Pro-NH-$C_2H_5$-tosylat

3,01 g (3 mmol) H-D-Trp-Ser($Ac_3$-D-Rib)-Arg-Pro-NH-$C_2H_5$-tosylat werden analog Beispiel 11f mit 2,03 g Fmoc-Trp-Ser-Tyr-OH umgesetzt.

Ausbeute 4,63; Schmp. 147-149 °C unter Zersetzung; $[\alpha]_D^{22}$ = -25,6° (c=1, in Methanol).

19g. H-Trp-Ser-Tyr-D-Trp-Ser($Ac_3$-D-Rib)-Arg-Pro-NH-$C_2H_5$-tosylat

4,15 g (2,5 mmol) Fmoc-Trp-Ser-Tyr-D-Trp-Ser($Ac_3$-D-Rib)-Arg-Pro-NH-$C_2H_5$-tosylat werden analog Beispiel 11g umgesetzt.

Ausbeute 3,75 g; Schmp. 99-102 °C unter Zersetzung; $[\alpha]_D^{22}$ = -27,2° (c=1, in Methanol).

19h. Pgl-His-Trp-Ser-Tyr-D-Trp-Ser(D-Rib)-Arg-Pro-NH-$C_2H_5$-acetat

1,44 g (1 mmol) H-Trp-Ser-Tyr-D-Trp-Ser($Ac_3$-D-Rib)-Arg-Pro-NH-$C_2H_5$-tosylat werden analog Beispiel 11h mit 450 mg Pgl-His(Dnp)-OH umgesetzt.

Ausbeute an Rohacetat: 975,8 mg;

Ausbeute nach der Reinigung: 495 mg; $[\alpha]_D^{22}$ = -60,6° (c=1, in Wasser).

20. Beispiel:

Pgl-His-Trp-Ser-Tyr-D-Trp-Ser(D-Ara)-Arg-Pro-NH-$C_2H_5$

20a. Fmoc-Ser($Ac_3$-D-Ara)-OBzl

Analog Beispiel 8a werden Fmoc-Ser-OBzl und 2,3,4-Tri-O-acetyl-D-arabinopyranosyl-bromid umgesetzt.

$[\alpha]_D^{22}$ = +8,9° (c=1, in Chloroform).

20b. Fmoc-Ser($Ac_3$-D-Ara)-OH

Fmoc-Ser($Ac_3$-D-Ara)-OBzl werden analog Beispiel 8b katalytisch hydriert.

$[\alpha]_D^{22}$ = +20,8° (c=1, in Chloroform).

20c. H-Ser($Ac_3$-D-Ara)-Arg-Pro-NH-$C_2H_5$-tosylat

4,1 g (7 mmol) Fmoc-Ser($Ac_3$-D-Ara)-OH werden analog Beispiel 1d mit 4,5 g H-Arg-Pro-NH-$C_2H_5$-ditosylatumgesetzt.

Der schaumige Rückstand wird analog Beispiel 6c umgesetzt.

Ausbeute 2,95 g; $[\alpha]_D^{22}$ = -29° (c=1, in Wasser).

20d. Fmoc-D-Trp-Ser($Ac_3$-D-Ara)-Arg-Pro-NH-$C_2H_5$-tosylat

2,45 g (3 mmol) H-Ser($Ac_3$-D-Ara)-Arg-Pro-NH-$C_2H_5$-tosylat werden analog Beispiel 6d mit 1,28 g Fmoc-D-Trp-OH umgesetzt.

Ausbeute 3,22 g; Schmp. 101-103 °C unter Zersetzung; $[\alpha]_D^{22}$ = -21,8° (c=1, in Methanol).

20e. H-D-Trp-Ser($Ac_3$-D-Ara)-Arg-Pro-NH-$C_2H_5$-tosylat

2,63 g (2,5 mmol) Fmoc-D-Trp-Ser($Ac_3$-D-Ara)-Arg-Pro-NH-$C_2H_5$-tosylat werden analog Beispiel 3d und 4c umgesetzt.
Ausbeute 2,19 g; Schmp. 102-105 °C unter Zersetzung; $[\alpha]_D^{22}$ = -14,3° (c=1, in Methanol).

20f. Fmoc-Trp-Ser-Tyr-D-Trp-Ser($Ac_3$-D-Ara)-Arg-Pro-NH-$C_2H_5$-tosylat

2,0 g (2 mmol) H-D-Trp-Ser($Ac_3$-D-Ara)-Arg-Pro-NH-$C_2H_5$-tosylat werden analog Beispiel 11f mit 1,35 g Fmoc-Trp-Ser-Tyr-OH umgesetzt.
Ausbeute 2,77 g; Schmp. 150-151 °C unter Zersetzung; $[\alpha]_D^{22}$ = -16,0° (c=1, in Methanol).

20g. H-Trp-Ser-Tyr-D-Trp-Ser($Ac_3$-D-Ara)-Arg-Pro-NH-$C_2H_5$-tosylat

2,45 g (1,5 mmol) Fmoc-Trp-Ser-Tyr-D-Trp-Ser($Ac_3$-D-Ara)-Arg-Pro-NH-$C_2H_5$-tosylat werden analog Beispiel 11 g umgesetzt.
Ausbeute 2,1 g; Schmp. 109-110 °C unter Zersetzung; $[\alpha]_D^{22}$ = -29,4° (c=1, in Methanol).

20h. Pgl-His-Trp-Ser-Tyr-D-Trp-Ser($Ac_3$-D-Ara)-Arg-Pro-NH-$C_2H_5$-acetat

1,44 g (1 mmol) H-Trp-Ser-Tyr-D-Trp-Ser($Ac_3$-D-Ara)-Arg-Pro-NH-$C_2H_5$-tosylat werden analog Beispiel 11h mit 450 mg Pgl-His(Dnp)-OH umgesetzt.
Ausbeute an Rohacetat: 1,033 g
Ausbeute nach chromatographischer Reinigung: 336 mg; $[\alpha]_D^{22}$ = -51,7° (c=1, in Wasser).

21. Beispiel:

Pgl-His-Trp-Ser-Tyr-D-Ser(D-Rib)-Leu-Arg-Pro-NH-$C_2H_5$

21a. Fmoc-D-Ser($Ac_3$-D-Rib)-OBzl

Analog Beispiel 8a werden Fmoc-D-Ser-OBzl und 2,3,4-Tri-O-acetyl-D-ribopyranosylbromid umgesetzt. $[\alpha]_D^{22}$ = -37,7° (c=1, in Chloroform).

21b. Fmoc-D-Ser($Ac_3$-D-Rib)-OH

Fmoc-D-Ser($Ac_3$-D-Rib)-OBzl werden analog Beispiel 8b katalytisch hydriert. $[\alpha]_D^{22}$ = -78,9° (c=1, in Chloroform).

21c. H-D-Ser($Ac_3$-D-Rib)-Leu-Arg-Pro-NH-$C_2H_5$-tosylat

4,1 g (7 mmol) Fmco-D-Ser($Ac_3$-D-Rib)-OH werden analog Beispiel 1d mit 5,3 g H-Leu-Arg-Pro-NH-$C_2H_5$-ditosylat umgesetzt.
Der schaumige Rückstand wird analog Beispiel 3d und 4e umgesetzt und gereinigt.
Ausbeute 6,26 g; $[\alpha]_D^{22}$ = -79,0° (c=1, in Wasser).

21d. Fmoc-Trp-Ser-Tyr-D-Ser($Ac_3$-D-Rib)-Leu-Arg-Pro-NH-$C_2H_5$-tosylat

2,79 g (3 mmol) H-D-Ser($Ac_3$-D-Rib)-Leu-Arg-Pro-NH-$C_2H_5$-tosylat werden analog Beispiel 11f mit 2,03 g Fmoc-Trp-Ser-Tyr-OH umgesetzt.
Ausbeute 4,22 g; Schmp. 144-146 °C unter Zersetzung; $[\alpha]_D^{22}$ = -44,1° (c=1, in Methanol).

21e. H-Trp-Ser-Tyr-D-Ser($Ac_3$-D-Rib)-Leu-Arg-Pro-NH-$C_2H_5$-tosylat

3,18 g (2 mmol) Fmoc-Trp-Ser-Tyr-D-Ser($Ac_3$-D-Rib)-Leu-Arg-Pro-NH-$C_2H_5$-tosylat werden analog Beispiel 11g umgesetzt.
Ausbeute 2,47 g; Schmp. 136-138 °C unter Zersetzung; $[\alpha]_D^{22}$ = -50,9° (c=1, Methanol).

21f. Pgl-His-Trp-Ser-Tyr-D-Ser(D-Rib)-Leu-Arg-Pro-NH-$C_2H_5$-acetat

1,19 g (1 mmol) H-Trp-Ser-Tyr-D-Ser($Ac_3$-D-Rib)-Leu-Arg-Pro-NH-$C_2H_5$-tosylat werden analog Beispiel 11h mit 450 g Pgl-His(Dnp)-OH umgesetzt.
Ausbeute an Rohacetat: 734,5 mg.
Ausbeute nach chromatographischer Reinigung: 349 mg; $[\alpha]_D^{22}$ = -51,9° (c=1, in Wasser).

22. Beispiel

Pgl-His-Trp-Ser-Tyr-D-Ser(D-Ara)-Leu-Arg-Pro-NH-$C_2H_5$

22a. Fmoc-D-Ser($Ac_3$-D-Ara)-OBzl

Analog Beispiel 8a werden Fmoc-D-Ser-OBzl und 2,4,5-Triacetyl-arabinopyranosyl-bromid umgesetzt.
$[\alpha]_D^{22}$ = -0,5° (c=1, in Chloroform).

22b. Fmoc-D-Ser($Ac_3$-D-Ara)-OH

Fmoc-D-Ser($Ac_3$-D-Ara)-OBzl werden analog Beispiel 8b katalytisch hydriert.
$[\alpha]_D^{22}$ = -5,8° (c=1, in Chloroform).

22c. H-D-Ser($Ac_3$-D-Ara)-Leu-Arg-Pro-NH-$C_2H_5$-tosylat

3,51 g (5 mmol) Fmoc-D-Ser($Ac_3$-D-Ara)-OH werden analog Beispiel 1d mit 4,54 g H-Leu-Arg-Pro-NH-$C_2H_5$-ditosylat umgesetzt.
Der schaumige Rückstand wird analog Beispiel 3d und 4e umgesetzt und gereinigt.
Ausbeute 5,47 g; $[\alpha]_D^{22}$ = -47,8° (c=1, in Wasser).

22d. Fmoc-Trp-Ser-Tyr-D-Ser($Ac_3$-D-Ara)-Leu-Arg-Pro-NH $C_2H_5$-tosylat

2,79 g (3 mmol) H-D-Ser($Ac_3$-D-Ara)-Leu-Arg-Pro-NH-$C_2H_5$-tosylat werden analog Beispiel 11f mit 2,03 g Fmoc-Trp-Ser-Tyr-OH umgesetzt.
Ausbeute 4,18 g; Schmp. 152-154° C unter Zersetzung; $[\alpha]_D^{22}$ = -30,4° (c=1, in Methanol).

22e. H-Trp-Ser-Tyr-D-Ser($Ac_3$-D-Ara)-Leu-Arg-Pro-NH-$C_2H_5$-tosylat

3,18 g (2 mmol) Fmoc-Trp-Ser-Tyr-D-Ser($Ac_3$-D-Ara)-Leu-Arg-Pro-NH-$C_2H_5$-tosylat werden analog Beispiel 11g umgesetzt.
Ausbeute 2,62 g; Schmp. 101-104° C unter Zersetzung; $[\alpha]_D^{22}$ = -31,6° (c=1, in Methanol).

22f. Pgl-His-Trp-Ser-Tyr-D-Ser(D-Ara)-Leu-Arg-Pro-NH-$C_2H_5$-acetat

1,19 g H-Trp-Ser-Tyr-D-Ser($Ac_3$-D-Ara)-Leu-Arg-Pro-NH-$C_2H_5$-tosylat werden analog Beispiel 11h mit 450 mg Pgl-His(Dnp)-OH umgesetzt.
Ausbeute an Rohacetat: 778,3 mg.
Ausbeute nach chromatographischer Reinigung: 365 mg; $[\alpha]_D^{22}$ = -32,6° (c=1, in Wasser).

23. Beispiel:

Pgl-His-Trp-Ser-Tyr-D-Ser(β-D-Glc-NAc)-Leu-Arg-Pro-NH-$C_2H_5$

23a. Fmoc-D-Ser($Ac_3$-β-D-Glc-NAc)-OBzl

Analog Beispiel 8a werden Fmoc-D-Ser-OBzl und N-Acetyl-3,4,6-Tri-O-acetyl-glucosamino-pyranosyl-bromid umgesetzt.
$[\alpha]_D^{22}$ = -11,0° (c=1, in Essigester).

23b. Fmoc-D-Ser($Ac_3$-β-D-Glc-NAc)-OH

Fmoc-D-Ser($Ac_3$-β-D-Glc-NAc)-OBzl werden analog Beispiel 8b katalytisch hydriert.

$[\alpha]_D^{22}$ = -23,8° (c = 1, in Essigester).

23c. H-D-Ser($Ac_3$-β-D-Glc-NAc)-Leu-Arg-Pro-NH-$C_2H_5$-tosylat

3,37 g (5 mmol) Fmoc-D-Ser($Ac_3$-β-D-Glc-NAc)-OH werden analog Beispiel 1d mit 3,75 g H-Leu-Arg-Pro-NH-$C_2H_5$-ditosylat umgesetzt.
Der schaumige Rückstand wird analog Beispiel 6c umgesetzt.
Ausbeute 3,96 g; $[\alpha]_D^{22}$ = -69,4° (c = 1, in Wasser).

23d. Fmoc-Trp-Ser-Tyr-D-Ser($Ac_3$-β-D-Glc-NAc)-Leu-Arg-Pro-NH-$C_2H_5$-tosylat

3,05 g (3 mmol) H-D-Ser($Ac_3$-β-Glc-NAc)-Leu-Arg-Pro-NH-$C_2H_5$-tosylat werden analog Beispiel 11f mit 2,03 g Fmoc-Trp-Ser-Tyr-OH umgesetzt.
Ausbeute 4,34 g; Schmp. 139-144° C unter Zersetzung; $[\alpha]_D^{22}$ = -36,8° (c = 1, in Methanol).

23e. H-Trp-Ser-Tyr-D-Ser($Ac_3$-β-Glc-NAc)-Leu-Arg-Pro-NH-$C_2H_5$-tosylat

3,35 g (2 mmol) Fmoc-Tyr-Ser-Tyr-D-Ser($Ac_3$-β-D-Glc-NAc)-Leu-Arg-Pro-NH-$C_2H_5$-tosylat werden analog Beispiel 11g umgesetzt.
Ausbeute 2,3 g; Schmp. 123-127° C unter Zersetzung; $[\alpha]_D^{22}$ = -40,2° (c = 1, in Methanol).

23f. Pgl-His-Trp-Ser-Tyr-D-Ser(β-D-Glc-NAc)-Leu-Arg-Pro-NH-$C_2H_5$-acetat

1,45 g (1 mmol) H-Trp-Ser-Tyr-D-Ser($Ac_3$-β-Glc-NAc)-Leu-Arg-Pro-NH-$C_2H_5$-tosylat werden analog Beispiel 11h mit 450 mg Pgl-His(Dnp)-OH umgesetzt.
Ausbeute an Rohacetat: 950 mg.
Ausbeute nach chromatographischer Reinigung: 353 mg; $[\alpha]_D^{22}$ = -53,1° (c = 1, in Wasser).

24. Beispiel:

Ac-D-Nal-p-Cl-D-Phe-D-Trp-Ser-Tyr-D-Ser(α-L-Rha)-Leu-Arg-Pro-Azagly-$NH_2$

24a. Fmoc-D-Trp-Ser(tBu)-OtBu

Zu einer Lösung von 17,1 g (40 mmol) Fmoc-D-Trp-OH, 10,12 g H-Ser(tBu)-OtBu•HCl und 5,4 g HOBt in 100 ml Dimethylformamid gibt man bei 0° C unter Rühren 5,2 ml N-Ethyl-morpholin und anschließend 8,8 g DCC. Man läßt 1 Stunde bei 0° C und 3 Stunden bei Raumtemperatur rühren. Danach wird der Niederschlag abgesaugt und das Filtrat im Vakuum eingeengt. Der Rückstand wird in Essigester gelöst und nacheinander mit Wasser, gesättigter $NaHCO_3$-Lösung, $KHSO_4/K_2SO_4$-Lösung, gesättigter $NaHCO_3$-Lösung und Wasser gewaschen, über $Na_2SO_4$ getrocknet und im Vakuum eingeengt. Die Lösung des resultierenden Öls in 70 ml Diethylether wird unter Rühren in 700 ml Petrolether getropft. Der Niederschlag wird abgesaugt und getrocknet.
Ausbeute 17,6 g; Schmp. 87-91° C; $[\alpha]_D^{22}$ = +22,3° (c = 1, in Methanol).

24b. Fmoc-p-Cl-D-Phe-D-Trp-Ser(tBu)-OtBu

Zu einer Lösung von 16,5 g (26,3 mmol) Fmoc-D-Trp-Ser(tBu)-OtBu in 150 ml Dimethylformamid gibt man bei Raumtemperatur 27,5 ml Diethylamin und läßt 45 Minuten bei Raumtemperatur stehen. Danach wird das Lösungsmittel im Vakuum abdestilliert und der Rückstand an Kieselgel zunächst in Methylenchlorid und danach in einer Mischung aus Methylenchlorid/Methanol wie 9:1 chromatographiert.
Ausbeute 7,95 g H-D-Trp-Ser(tBu)-OtBu.
Zu einer Lösung des oben gewonnenen Öls (19,7 mmol), 8,2 g Fmoc-p-Cl-D-Phe-OH und 2,6 g HOBt in 100 ml Dimethylformamid gibt man 4,3 DCC. Man arbeitet wie in Beispiel 24 a auf. Der Rückstand kristallisiert aus Diethylether.
Ausbeute 10,2 g; Schmp. 184-186°; $[\alpha]_D^{23}$ = +14,8° (c = 1, in Methanol).

24c. Ac-D-Nal-p-Cl-D-Phe-D-Trp-Ser(tBu)-OtBu

Zu einer Lösung von 10 g (12,38 mmol) Fmoc-p-Cl-D-Phe-D-Trp-Ser(tBu)-OtBu in 100 ml Dimethylformamid gibt man 24,6 ml Diethylamin und läßt 20 Minuten bei Raumtemperatur stehen. Nach Abdestillieren des Lösungsmittels wird an Kieselgel in Methylenchlorid chromatographiert. Ausbeute 7,2 g Öl.

Zu einer Lösung des oben gewonnenen Öls (12,3 mmol), 2,94 g Ac-D-Nal-OH, 2,04 g HOObt in 90 ml Dimethylformamid gibt man bei 0° C 2,7 g DCC. Man arbeitet wie bei Beispiel 24a auf. Der Rückstand wird mit Diethylether verrieben, abgesaugt, in 18 ml Methanol warm gelöst und mit 230 ml Diethylether gefällt. Der Niederschlag wird nach Kühlung abgesaugt und getrocknet.

Ausbeute 6,4 g; Schmp. 204-207° C; $[\alpha]_D^{22}$ = -20,5° (c=1, in 80 %iger Essigsäure).

24d. Ac-D-Nal-P-Cl-D-Phe-D-Trp-Ser-OH

Zu einer Lösung aus 60 ml 90 %iger wäßriger Trifluoressigsäure und 15 ml 1,2-Ethandithiol gibt man bei Raumtemperatur unter Rühren 6,2 g Ac-D-Nal-p-Cl-D-Phe-D-Trp-Ser(tBu)-OtBu. Man läßt 75 Minuten bei Raumtemperatur rühren und engt im Vakuum ein. Der Rückstand wird mit Wasser verrieben, abgesaugt und über $P_2O_5$ getrocknet. Die Substanz wird aus Isopropanol/Petrolether umgefällt.

Ausbeute 4,21 g; Schmp. 195-197° C; $[\alpha]_D^{22}$ = -11,5° (c=1, in 80 %iger Essigsäure).

24e. Z-Pro-Azagly-$NH_2$

Zu einer Lösung vonn 125 g (500 mmol) Z-Pro-OH, 55 g Semicarbazid-hydrochlorid und 67,5 g HOBt in 1000 ml Dimethylformamid gibt man bei 0° C unter Rühren 75 ml Triethylamin und 105 g DCC. Man läßt etwa einen Tag bei 4° C reagieren und saugt den Niederschlag ab. Das Filtrat wird eingeengt und der Rückstand mit gesättigter $NaHCO_3$-Lösung verrieben. Der Niederschlag wird abgesaugt, gut mit Wasser gewaschen und über $P_2O_5$ im Vakuum getrocknet.

Ausbeute 135,3 g; Schmp. 189° C.

24f. Z-Arg($Z_2$)-Pro-Azagly-$NH_2$

131,6 g (430 mmol) Z-Pro-Azagly-$NH_2$ werden in 1000 ml einer Mischung von Methanol und Dimethylformamid (1:1) analog Beispiel 1e katalytisch hydriert.

Der Rückstand wird mit Wasser verrührt. Unlösliches wird abgesaugt und verworfen. Das Filtrat wird eingeengt.

Ausbeute 85,2 g H-Pro-Azagly-$NH_2$•Cl.

Zu einer Lösung von 20,8 g (100 mmol) der oben gewonnenen Substanz, 57,6 g Z-Arg($Z_2$)-OH und 16,3 g HOObt in 400 ml Dimethylformamid gibt man unter Rühren bei 0° C 21 g DCC. Man arbeitet analog Beispiel 24a auf. Man verreibt den Rückstand mit Diethylether, dekantiert ab und verreibt erneut mit Petrolether.

Ausbeute 73,4 g; $[\alpha]_D^{20}$ = -28,4° (c=1, in Methanol).

24g. H-Arg-Pro-Azagly-$NH_2$•HCl

37,5 g Z-Arg($Z_2$)-Pro-Azagly-$NH_2$ werden analog Beispiel 1e in 350 ml Methanol katalytisch hydriert. Der Rückstand wird mit Diethylether verrieben, abgesaugt und getrocknet.

Ausbeute 21,95 g; $[\alpha]_D^{21}$ = -11,4° (c=1, in Methanol).

24h. H-Leu-Arg-Pro-Azagly-$NH_2$•2HCl

13,2 g (30 mmol) H-Arg-Pro-Azagly-$NH_2$•2HCl werden analog Beispiel 1d mit 7,92 g Z-Leu-OH umgesetzt.

Ausbeute 16,7 g Z-Leu-Arg-Pro-Azagly-$NH_2$•HCl; $[\alpha]_D^{22}$ = -45,8° (c=1, in 80 %iger Essigsäure).

14,5 g der oben gewonnenen Substanz werden analog Beispiel 1e in 150 ml Methanol katalytisch hydriert. Der Rückstand wird mit Diethylether verrieben.

Ausbeute 12,35 g; $[\alpha]_D^{22}$ = -26,4° (c=1, in Methanol).

24i. Fmoc-D-Ser($Ac_3$-α-L-Rha)-Leu-Arg-Pro-Azagly-$NH_2$•HCl

10,43 g (17,4 mmol) Fmoc-D-Ser($Ac_3$-α-L-Rha)-OH werden analog Beispiel 1d mit 8,95 g H-Leu-Arg-Pro-Azagly-$NH_2$•2HCl umgesetzt.

Ausbeute 15,07 g; $[\alpha]_D^{21}$ = -48,4° (c=1, in Wasser).

24k. H-D-Ser($Ac_3$-α-L-Rha)-Leu-Arg-Pro-Azagly-$NH_2$•HCl

Zu einer Lösung von 14,3 g (13,5 mmol) Fmoc-D-Ser($Ac_3$-α-L-Rha)-Leu-Arg-Pro-Azagly-$NH_2$•HCl in 65 ml Dimethylformamid gibt man 14,3 ml (135 mmol) Diethylamin und rührt 10 Minuten bei Raumtemperatur. Danach engt man im Vakuum ein und verrührt den Rückstand mit Diethylether.

Ausbeute 12,8 g.

Zur Reinigung verrührt man die Substanz mit 1000 ml Wasser, saugt von Umgelöstem ab und gefriertrocknet das Filtrat.

Ausbeute 10,75 g; $[\alpha]_D^{22}$ = -67,4° (c=1, in Methanol).

24l. Fmoc-Tyr-D-Ser($Ac_3$-α-L-Rha)-Leu-Arg-Pro-Azagly-$NH_2$•HCl

3,35 g (4 mmol) H-D-Ser($Ac_3$-α-L-Rha)-Leu-Arg-Pro-Azagly-$NH_2$•HCl werden analog Beispiel 11f mit 1,62 g Fmoc-Tyr-OH umgesetzt. Die Pentanol-Phase wird noch mit $NaHCO_3$-Lösung ausgeschüttelt, anschließend mit 1N HCl auf pH 7 gestellt und eingeengt. Der Rückstand wird mit Diethylether verrieben.

Ausbeute 4,2 g; $[\alpha]_D^{22}$ = -35,0° (c=1, in Methanol).

24m. H-Tyr-D-Ser($Ac_3$-α-L-Rha)-Leu-Arg-Pro-Azagly-$NH_2$•HCl

3,67 g (3 mmol) Fmoc-Tyr-D-Ser($Ac_3$-α-L-Rha)-Leu-Arg-Pro-Azagly-$NH_2$ werden analog Beispiel 24k umgesetzt. Zur Reinigung verteilt man in 3 Stufen zwischen p-Pentanol und Wasser. Die wäßrigen Phasen und die n-Pentanolphasen 2 - 3 werden vereinigt, eingeengt und der Rückstand mit Diethylether verrieben.

Ausbeute 2,3 g; $[\alpha]_D^{21}$ = -53,2° (c=1, in Methanol).

24n. Ac-D-Nal-p-Cl-D-Phe-D-Trp-Ser-Tyr-D-Ser(α-L-Rha)-Leu-Arg-Pro-Azagly-$NH_2$-acetat

712 mg (1 mmol) Ac-D-Nal-p-Cl-D-Phe-D-Trp-Ser-OH werden analog Beispiel 11h mit 1 g H-Tyr-D-Ser-($Ac_3$-α-L-Rha)-Leu-Arg-Pro-Azagly-$NH_2$•HCl umgesetzt.

Ausbeute an Rohacetat: 897 mg.

Ausbeute nach chromatographischer Reinigung: 214 mg; $[\alpha]_D^{23}$ = +232,9° (c=1, in Wasser).

25. Beispiel:

Ac-D-Nal-p-Cl-D-Phe-D-Trp-Ser-His-D-Ser(α-L-Rha)-Leu-Arg-Pro-Azagly-$NH_2$

25a. Fmoc-His(Dnp)-D-Ser($Ac_3$-α-L-Rha)-Leu-Arg-Pro-Azagly-$NH_2$•HCl

3,35 g (4 mmol) H-D-Ser($Ac_3$-α-L-Rha)-Leu-Arg-Pro-Azagly-$NH_2$•HCl werden analog Beispiel 241 mit 2,17 g Fmoc-His(Dnp)-OH umgesetzt.

Ausbeute 4,9 g; $[\alpha]_D^{22}$ = -31,9° (c=1, in Methanol).

25b. H-His-D-Ser(α-L-Rha)-Leu-Arg-Pro-Azagly-$NH_2$•HCl

Zu einer Lösung von 4,1 g Fmoc-His(Dnp)-D-Ser($Ac_3$-α-L-Rha)-Leu-Arg-Pro-Azagly-$NH_2$•HCl in 15 ml Dimethylacetamid gibt man 3 ml 100 %iges Hydrazinhydrat und rührt 4 Stunden bei Raumtemperatur. Danach wird im Vakuum eingeengt und der Rückstand mit Diethylether verrührt und abgesaugt. Danach wird die Substanz in wenig Methanol gelöst, von Unlöslichem filtriert und mit Essigester gefällt.

Ausbeute 2,25 g, $[\alpha]_D^{22}$ = -57,4° (c=1, in Methanol).

25c. Ac-D-Nal-p-Cl-D-Phe-D-Trp-Ser-His-D-Ser(α-L-Rha)-Leu-Arg-Pro-Azagly-$NH_2$-acetat

712 mg (1 mmol) Ac-D-Nal-p-Cl-D-Phe-D-Trp-Ser-OH werden analog Beispiel 3e mit 850 mgl H-His-D-Ser(α-L-Rha)-Leu-Arg-Pro-Azagly-$NH_2$•HCl umgesetzt. Der Rückstand wird mit Essigester verrieben und in ca. 100-150 ml 30 %iger Essigsäure gelöst. Von Unlöslichem wird filtriert und das Filtrat über einen schwach basichen Ionenaustauscher in Acetat-Form chromatographiert.

Ausbeute an Rohacetat: 1,4 g.

Ausbeute nach chromatographischer Reinigung: 538 mg; $[\alpha]_D^{24}$ = -213,1° (c=1, in Wasser).

26. Beispiel

Ac-D-Nal-p-Cl-D-Phe-D-Trp-Ser-Arg-D-Ser(α-L-Rha)-Leu-Arg-Pro-Azagly-$NH_2$

26a. Fmoc-Arg-D-Ser($Ac_3$-α-L-Rha)-Leu-Arg-Pro-Azagly-$NH_2$•2HCl

Zu einer Lösung von 1,59 g (4 mmol) Fmoc-Arg-OH, 3,35 g H-D-Ser($Ac_3$-α-L-Rha)-Leu-Arg-Pro-Azagly-$NH_2$•HCl, 540 mg HOBt und 720 mg Pyridiniumperchlorat in 20 ml Dimethylformamid gibt man unter Rühren bei 0°C 880 mg DCC. Man rührt 1 Stunde bei 0°C und 3 Stunden bei Raumtemperatur. Man läßt über Nacht stehen und saugt den Niederschlag ab. Das Filtrat wird im Vakuum eingeengt und der Rückstand zwischen n-Pentanol und $NaHCO_3$ verteilt. Die Pentanolphase wird nochmals mit $NaHCO_3$-Lösung und Wasser extrahiert, mit 1 n HCl auf pH 7 eingestellt und eingeengt. Der Rückstand wird mit Diethylether verrieben und abgesaugt.

Ausbeute 4,3 g; $[\alpha]_D^{22}$ = -37,1° (c=1, in Methanol).

26b. H-Arg-D-Ser($Ac_3$-α-L-Rha)-Leu-Arg-Pro-Azagly-$NH_2$•2HCl

3,77 g (3 mmol) Fmoc-Arg-D-Ser($Ac_3$-α-L-Rha)-Leu-Arg-Pro-Azagly-$NH_2$•2HCl werden analog Beispiel 24k umgesetzt. Gereinigt wird durch eine 3-stufige Gegenstromverteilung zwischen n-Pentanol und Wasser. Die Wasserphasen 1 - 2 werden vereinigt und gefriergetrocknet.

Ausbeute 2,4 g; $[\alpha]_D^{21}$ = -48,2° (c=1, in Methanol).

26c. Ac-D-Nal-p-Cl-D-Phe-D-Trp-Ser-Arg-D-Ser(α-L-Rha)-Leu Arg-Pro-Azagly-$NH_2$-diacetat

712 mg (1 mmol) Ac-D-Nal-p-Cl-D-Phe-D-Trp-Ser-OH werden analog Beispiel 11h mit 1,03 H-Arg-D-Ser($Ac_3$-α-L-Rha)-Leu-Arg-Pro-Azagly-$NH_2$•2HCl umgesetzt.

Ausbeute an Rohacetat: 1,06 g.

Ausbeute nach chromographischer Reinigung: 550 mg; $[\alpha]_D^{23}$ = -60,8° (c=1, in Wasser).

**Patentansprüche**

**Patentansprüche für folgende Vertragsstaaten : AT, BE, CH, DE, FR, GB, GR, IT, LI, LU, NL, SE**

**1.** Peptid der allgemeinen Formel I

```
1 2 3 4   5  6 7 8  9   10
X-A-B-C-Ser-D-E-F-Arg-Pro-G          (I),
```

in welcher

- X Wasserstoff oder ($C_1$-$C_7$)-Acyl bedeutet oder, falls A für Pyroglutamyl steht, abwesend ist;
- A Pgl, dehydro-Pro, Pro-, D-Thi, D-Pgl oder gegebenenfalls durch einen oder zwei gleiche oder verschiedene Reste aus der Reihe Brom, Chlor, Fluor, Nitro, Amino, Methyl und Methoxy im Aromaten substituiertes D-Nal(2), in dieser Weise substituiertes D-Phe oder in dieser Weise substituiertes D-Trp bedeutet;
- B His oder gegebenenfalls durch einen oder zwei gleiche oder verschiedene Reste aus der Reihe Brom, Chlor, Fluor, Nitro, Amino, Methyl und Methoxy im Phenylring substituiertes D-Phe bedeutet;
- C Trp, D-Thi, D-Pal(3) oder gegebenenfalls in Position 5 und/oder 6 durch einen oder zwei gleiche oder verschiedene Reste aus der Reihe Brom, Chlor, Fluor, Nitro, Amino, Methyl oder Methoxy substituiertes D-Trp bedeutet;
- D Tyr, Arg oder His bedeutet;
- E D-Ser($R^1$), β-Asn, β-Asp-OMe, D-Thi oder den Rest einer D-Aminosäure der allgemeinen Formel II bedeutet;

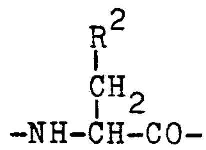

(II)

F Ser($R^1$), Leu, Trp oder Phe bedeutet;
G Gly-$NH_2$, Aza-Gly-$NH_2$, D-Ala-$NH_2$ oder NH-($C_1$-$C_4$)-alkyl bedeutet
$R^1$ einen gegebenenfalls teilgeschützten Glycosylrest mit wenigstens einer freien Hydroxygruppe bedeutet und
$R^2$ Wasserstoff, ($C_1$-$C_4$)-Alkoxycarbonyl, ($C_1$-$C_4$)-Alkoxy, ($C_1$-$C_4$)-Alkyl, das gegebenenfalls durch ($C_1$-$C_4$)-Alkoxycarbonyl oder ($C_1$-$C_4$)-Alkoxycarbonylamino monosubstituiert ist, Phenyl, das gegebenenfalls durch bis zu drei gleiche oder verschiedene Reste der aus Reihe Chlor, Fluor, Methyl und ($C_1$-$C_4$)-Alkoxy substituiert ist, Naphthyl, 4,5,6,7-Tetrahydrobenzimidazol-2-yl oder Indolyl bedeutet,
oder dessen physiologisch verträgliches Salz, mit der Maßgabe, daß

a) falls E für einen Rest der Formel II, β-Asn, β-Asp-OMe oder D-Thi steht, F ausschließlich die Bedeutung Ser($R^1$) zukommt und
b) falls F für Leu, Phe oder Trp steht, E ausschließlich die Bedeutung D-Ser($R^1$) zukommt.

**2.** Peptid der allgemeinen Formel I gemäß Anspruch 1, in welcher

X abwesend ist;
A Pgl;
B His;
C Trp;
D Tyr oder His;
E D-Ser($R^1$), β-Asn, β-Asp-OMe oder den Rest einer D-Aminosäure der Formel II;
F Ser($R^1$), Trp oder Leu und
G Gly-$NH_2$, Aza-Gly-$NH_2$ oder NH-($C_1$-$C_4$)-Alkyl
bedeuten und
$R^1$ und $R^2$ wie im Anpruch 1 definiert sind, oder dessen physiologisch verträgliches Salz.

**3.** Peptid der allgemeinen Formel I gemäß Anspruch 1, in welcher

X Wasserstoff oder ($C_1$-$C_7$)-Acyl bedeutet oder abwesend ist,
A dehydro-Pro, Pro, D-Thi, D-Pgl, gegebenenfalls substituiertes D-Nal(2), gegebenenfalls substituiertes D-Phe oder gegebenenfalls substiutieres D-Trp;
B gegebenenfalls substituiertes D-Phe;
C gegebenenfalls substituiertes D-Trp, D-Thi oder D-Pal(3);
D Tyr, Arg oder His;
E D-Ser($R^1$), D-Thi oder den Rest einer D-Aminosäure der Formel II;
F Ser($R^1$), Leu, Phe oder Trp und
G Gly-$NH_2$, D-Ala-$NH_2$, Aza-Gly-$NH_2$ oder NH-($C_1$-$C_4$)-Alkyl bedeuten und
$R^1$ und $R^2$ wie im Anspruch 1 definiert sind;
oder deren physiologisch verträgliches Salz.

**4.** Peptid der allgemeinen Formel I gemäß Anspruch 3, in welcher

X ($C_1$-$C_7$)-Acyl,
A D-Nal(2);
B D-Phe(Cl);
C D-Trp;
D Tyr, His oder Arg;
E D-Ser($R^1$);

F Ser($R^1$), Leu, Phe oder Trp und
G D-Ala-$NH_2$ oder Aza-Gly-$NH_2$ bedeuten und
$R^1$ und $R^2$ wie im Anspruch 1 definiert sind, oder dessen physiologisch verträgliches Salz.

**5.** Peptid der allgemeinen Formel I gemäß einem der Ansprüche 1 - 4, in welcher $R^1$ für einen ungeschützten Glycosylrest steht, oder dessen physiologisch verträgliches Salz.

**6.** Verfahren zur Herstellung eines Peptids gemäß einem der Ansprüche 1 - 5, dadurch gekennzeichnet, daß man ein Fragment mit N-terminaler freier Aminogruppe kondensiert mit einem Fragment mit C-terminaler freier Carboxylgruppe, eine oder mehrere zum Schutz funktioneller Gruppen gegebenenfalls temporär eingeführte Schutzgruppen abspaltet und das so erhaltene Peptid gegebenenfalls in sein physiologisch verträgliches Salz überführt.

**7.** Peptid gemäß einem der Ansprüche 1 - 4 zur Anwendung als Heilmittel.

**8.** Peptid gemäß einem der Ansprüche 1 - 4 zur Anwendung als fertilitätssteigerndes Mittel.

**9.** Peptid gemäß einem der Ansprüche 1 - 4 zur Anwendung als Plasma-Gonadotropin, -Testosteron und -Östrogen senkendes Mittel.

**10.** Pharmazeutische Zubereitung enthaltend ein Peptid gemäß einem der Ansprüche 1 - 4 oder dessen physiologisch verträgliches Salz.

**11.** Verfahren zur Herstellung einer Zubereitung gemäß Anspruch 10, dadurch gekennzeichnet, daß man ein Peptid der allgemeinen Formel I oder dessen physiologisch verträgliches Salz zusammen mit einem physiologisch annehmbaren Träger und gegebenenfalls weiteren Zusatz-, Hilfs- und/oder Konservierungsstoffen in eine geeignete Darreichungsform bringt.

**Patentansprüche für folgenden Vertragsstaat : ES**

**1.** Verfahren zur Herstellung eines Peptids der allgemeinen Formel I

```
1 2 3 4   5 6 7 8   9  10
X-A-B-C-Ser-D-E-F-Arg-Pro-G          (I),
```

in welcher

X Wasserstoff oder ($C_1$-$C_7$)-Acyl bedeutet oder, falls
A für Pyroglutamyl steht, abwesend ist; A Pgl, dehydro-Pro, Pro-, D-Thi, D-Pgl oder gegebenenfalls durch einen oder zwei gleicher oder verschiendene Reste aus der Reihe Brom, Chlor, Fluor, Nitro, Amino, Methyl und Methoxy im Aromaten substituiertes D-Nal(2), in dieser Weise substituiertes D-Phe oder in dieser Weise substituiertes D-Trp bedeutet;
B His oder gegebenenfalls durch einen oder zwei gleiche oder verschiedene Reste aus der Reihe Brom, Chlor, Fluor, Nitro, Amino, Methyl und Methoxy im Phenylring substituiertes D-Phe bedeutet;
C Trp, D-Thi, D-Pal(3) oder gegebenenfalls in Position 5 und/oder 6 durch einen oder zwei gleiche oder verschiedene Reste aus der Reihe Brom, Chlor, Fluor, Nitro, Amino, Methyl oder Methoxy substituiertes D-Trp bedeutet;
D Tyr, Arg oder His bedeutet;
E D-Ser($R^1$), $\beta$-Asn, $\beta$-Asp-OMe, D-Thi oder den Rest einer D-Aminosäure der allgemeinen Formel II bedeutet;

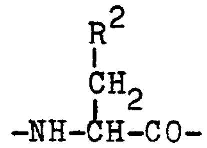

(II)

F Ser($R^1$), Leu, Trp oder Phe bedeutet;
G Gly-$NH_2$, Aza-Gly-$NH_2$, D-Ala-$NH_2$ oder NH-($C_1$-$C_4$)-alkyl bedeutet
$R^1$ einen gegebenenfalls teilgeschützten Glycosylrest mit wenigstens einer freien Hydroxygruppe bedeutet und
$R^2$ Wasserstoff, ($C_1$-$C_4$)-Alkoxycarbonyl, ($C_1$-$C_4$)-Alkoxy, ($C_1$-$C_4$)-Alkyl, das gegebenenfalls durch ($C_1$-$C_4$)-Alkoxycarbonyl oder ($C_1$-$C_4$)-Alkoxycarbonylamino monosubstituiert ist, Phenyl, das gegebenenfalls durch bis zu drei gleiche oder verschiedene Reste der aus Reihe Chlor, Fluor, Methyl und ($C_1$-$C_4$)-Alkoxy substituiert ist, Naphthyl, 4,5,6,7-Tetrahydrobenzimidazol-2-yl oder Indolyl bedeutet,
oder dessen physiologisch verträgliches Salz, mit der Maßgabe, daß

a) falls E für einen Rest der Formel II, $\beta$-Asn, $\beta$-Asp-OMe oder D-Thi steht, F ausschließlich die Bedeutung Ser($R^1$) zukommt und
b) falls F für Leu, Phe oder Trp steht, E ausschließlich die Bedeutung D-Ser($R^1$) zukommt,

dadurch gekennzeichnet, daß man ein Fragment mit N-terminaler freier Aminogruppe kondensiert mit einem Fragment mit C-terminaler freier Carboxylgruppe, eine oder mehrere zum Schutz funktioneller Gruppen gegebenenfalls temporär eingeführte Schutzgruppen abspaltet und das so erhaltene Peptid gegebenenfalls in sein physiologisch verträgliches Salz überführt.

**2.** Verfahren gemäß Anspruch 1, dadurch gekennzeichnet, daß man ein Peptid der allgemeinen Formel I herstellt, in welcher

X abwesend ist;
A Pgl;
B His;
C Trp;
D Tyr oder His;
E D-Ser($R^1$), $\beta$-Asn, $\beta$-Asp-OMe oder den Rest einer D-Aminosäure der Formel II;
F Ser($R^1$), Trp oder Leu und
G Gly-$NH_2$, Aza-Gly-$NH_2$ oder NH-($C_1$-$C_4$)-Alkyl
bedeuten und
$R^1$ und $R^2$ wie im Anpruch 1 definiert sind, oder dessen physiologisch verträgliches Salz.

**3.** Verfahren gemäß Anspruch 1, dadurch gekennzeichnet, daß man ein Peptid der allgemeinen Formel I herstellt, in welcher

X Wasserstoff oder ($C_1$-$C_7$)Acyl bedeutet oder abwesend ist,
A dehydro-Pro, Pro, D-Thi, D-Pgl, gegebenenfalls substituiertes D-Nal(2), gegebenenfalls substituiertes D-Phe oder gegebenenfalls substituieres D-Trp;
B gegebenenfalls substituiertes D-Phe;
C gegebenenfalls substituiertes D-Trp, D-Thi oder D-Pal(3);
D Tyr, Arg oder His;
E D-Ser($R^1$), D-Thi oder den Rest einer D-Aminosäure der Formel II;
F Ser($R^1$), Leu, Phe oder Trp und
G Gly-$NH_2$, D-Ala-$NH_2$, Aza-Gly-$NH_2$ oder NH-($C_1$-$C_4$)-Alkyl bedeuten und
$R^1$ und $R^2$ wie im Anspruch 1 definiert sind;
oder deren physiologisch verträgliches Salz.

**4.** Verfahren gemäß Anspruch 3, dadurch gekennzeichnet, daß man ein Peptid der allgemeinen Formel I

herstellt, in welcher

- X (C$_1$-C$_7$)-Acyl,
- A D-Nal(2);
- B D-Phe(Cl);
- C D-Trp;
- D Tyr, His oder Arg;
- E D-Ser($R^1$);
- F Ser($R^1$), Leu, Phe oder Trp und
- G D-Ala-$NH_2$ oder Aza-Gly-$NH_2$ bedeuten und

$R^1$ und $R^2$ wie im Anspruch 1 definiert sind, oder dessen physiologisch verträgliches Salz.

**5.** Verfahren gemäß einem der Ansprüche 1 - 4, dadurch gekennzeichnet, daß ein Peptid der allgemeinen Formel I hergestellt wird, in welcher $R^1$ für einen umgeschützten Glycosylrest steht, oder dessen physiologisch verträgliches Salz.

**6.** Verfahren zur Herstellung eines pharmazeutischen Mittels enthaltend ein Peptid der allgemeinen Formel I gemäß einem der Ansprüche 1 -4 , dadurch gekennzeichnet, daß man dieses zusammen mit einem physiologisch annehmbaren Träger und gegebenenfalls weiteren Zusatz-, Hilfs-und/oder Konservierungsstoffen in eine geeignete Darreichungsform bringt.

**Claims**

**Claims for the following Contracting States : AT, BE, CH, DE, FR, GB, GR, IT, LI, LU, NL, SE**

**1.** A peptide of the formula I

```
1 2 3  4   5 6 7  8   9  10
X-A-B-C-Ser-D-E-F-Arg-Pro-G          (I),
```

in which

- X is hydrogen or (C$_1$-C$_7$)-acyl or, if A represents pyroglutamyl, is absent;
- A is Pgl, dehydro-Pro, Pro, D-Thi, D-Pgl or D-Nal(2) optionally substituted in the aromatic ring by one or two identical or different radicals from the series bromo, chloro, fluoro, nitro, amino, methyl and methoxy, D-Phe substituted in this way or D-Trp substituted in this way;
- B is His or D-Phe optionally substituted in the phenyl ring by one or two identical or different radicals from the series bromo, chloro, fluoro, nitro, amino, methyl and methoxy;
- C is Trp, D-Thi, D-Pal(3) or D-Trp optionally substituted in position 5 and/or 6 by one or two identical or different radicals from the series bromo, chloro, fluoro, nitro, amino, methyl or methoxy;
- D is Tyr, Arg or His;
- E is D-Ser($R^1$), $\beta$-Asn, $\beta$-Asp-OMe, D-Thi or the radical of a D-amino acid of the formula II;

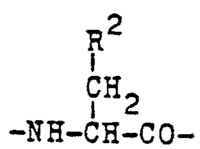

(II)

- F is Ser($R^1$), Leu, Trp or Phe;
- G is Gly-$NH_2$, Asa-Gly-$NH_2$, D-Ala-$NH_2$ or NH-(C$_1$-C$_4$)-alkyl;
- $R^1$ is an optionally partly protected glycosyl radical with at least one free hydroxyl group and
- $R^2$ is hydrogen, (C$_1$-C$_4$)-alkoxycarbonyl, (C$_1$-C$_4$)-alkoxy, (C$_1$-C$_4$)-alkyl which is optionally monosubstituted by (C$_1$-C$_4$)-alkoxycarbonyl or (C$_1$-C$_4$)-alkoxycarbonyl-amino, phenyl which is

optionally substituted by up to three identical or different radicals from the series chloro, fluoro, methyl and ($C_1$-$C_4$)-alkoxy, naphthyl, 4,5,6,7-tetrahydrobenzimidazol-2-yl or indolyl, or a physiologically acceptable salt thereof, with the proviso that

a) if E represents a residue of the formula II, β-Asn, β-Asp-OMe or D-Thi, F is exclusively Ser($R^1$) and

b) if F represents Leu, Phe or Trp, E is exclusively D-Ser($R^1$).

**2.** A peptide of the formula I as claimed in claim 1, wherein

X is absent;
A is Pgl;
B is His;
C is Trp;
D is Tyr or His;
E is D-Ser($R^1$), β-Asn, β-Asp-OMe or the residue of a D-amino acid of the formula II;
F is Ser($R^1$), Trp or Leu and
G is Gly-$NH_2$, Aza-Gly-$NH_2$ or NH-($C_1$-$C_4$)-alkyl and $R^1$ and $R^2$ are as defined in claim 1, or a physiologically acceptable salt thereof.

**3.** A peptide of the formula I as claimed in claim 1, wherein

X is hydrogen or ($C_1$-$C_7$)-acyl or is absent,
A is dehydro-Pro, Pro, D-Thi, D-Pgl, optionally substituted D-Nal(2), optionally substituted D-Phe or optionally substituted D-Trp;
B is optionally substituted D-Phe;
C is optionally substituted D-Trp, D-Thi or D-Pal(3);
D is Tyr, Arg or His;
E is D-Ser($R^1$), D-Thi or the radical of a D-amino acid of the formula II;
F is Ser($R^1$), Leu, Phe or Trp and
G is Gly-$NH_2$, D-Ala-$NH_2$, Aza-Gly-$NH_2$ or NH-($C_1$-$C_4$)-alkyl and
$R^1$ and $R^2$ are as defined in claim 1;

or a physiologically acceptable salt thereof.

**4.** A peptide of the formula I as claimed in claim 3, wherein

X is ($C_1$-$C_7$)-acyl,
A is D-Nal(2);
B is D-Phe(Cl);
C is D-Trp;
D is Tyr, His or Arg;
E is D-Ser($R^1$);
F is Ser($R^1$), Leu, Phe or Trp and
G is D-Ala-$NH_2$ or Aza-Gly-$NH_2$ and
$R^1$ and $R^2$ are as defined in claim 1,

or a physiologically acceptable salt thereof.

**5.** A peptide of the formula I as claimed in any of claims 1 to 4, wherein $R^1$ represents an unprotected glycosyl residue, or a physiologically acceptable salt thereof.

**6.** A process for the preparation of a peptide as claimed in any of claims 1 to 5, which comprises condensing a fragment with an N-terminal free amino group with a fragment with a C-terminal free carboxyl group, removing one or more protective groups optionally introduced temporarily to protect functional groups and optionally converting the peptide thus obtained into its physiologically acceptable salt.

**7.** A peptide as claimed in any of claims 1 to 4 for use as a medicament.

**8.** A peptide as claimed in any of claims 1 to 4 for use as an agent for increasing fertility.

**9.** A peptide as claimed in any of claims 1 to 4 for use as an agent for lowering plasma gonadotropin, testosterone and estrogen.

**10.** A pharmaceutical composition containing a peptide as claimed in any of claims 1 to 4 or a physiologically acceptable salt thereof.

**11.** A process for the preparation of a composition as claimed in claim 10, which comprises a peptide of the formula I or a physiologically acceptable salt thereof bringing into a suitable form for administration together with a physiologically acceptable carrier and optionally further additives, auxiliaries and/or preservatives.

**Claims for the following Contracting State : ES**

**1.** A process for the preparation of a peptide of the formula I

```
1 2 3   4   5 6 7  8    9   10
X-A-B-C-Ser-D-E-F-Arg-Pro-G          (I),
```

in which

X is hydrogen or ($C_1$-$C_7$)-acyl or, if A represents pyroglutamyl, is absent;

A is Pgl, dehydro-Pro, Pro, D-Thi, D-Pgl or D-Nal(2) optionally substituted in the aromatic ring by one or two identical or different radicals from the series bromo, chloro, fluoro, nitro, amino, methyl and methoxy, D-Phe substituted in this way or D-Trp substituted in this way;

B is His or D-Phe optionally substituted in the phenyl ring by one or two identical or different radicals from the series bromo, chloro, fluoro, nitro, amino, methyl and methoxy;

C is Trp, D-Thi, D-Pal(3) or D-Trp optionally substituted in position 5 and/or 6 by one or two identical or different radicals from the series bromo, chloro, fluoro, nitro, amino, methyl or methoxy;

D is Tyr, Arg or His;

E is D-Ser($R^1$), $\beta$-Asn, $\beta$-Asp-OMe, D-Thi or the radical of a D-amino acid of the formula II;

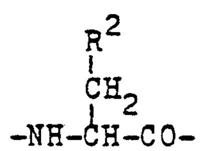

(II)

F is Ser($R^1$), Leu, Trp or Phe;

G is Gly-$NH_2$, Aza-Gly-$NH_2$, D-Ala-$NH_2$ or NH-($C_1$-$C_4$)-alkyl;

$R^1$ is an optionally partly protected glycosyl radical with at least one free hydroxyl group and

$R^2$ is hydrogen, ($C_1$-$C_4$)-alkoxycarbonyl, ($C_1$-$C_4$)-alkoxy, ($C_1$-$C_4$)-alkyl which is optionally monosubstituted by ($C_1$-$C_4$)-alkoxycarbonyl or ($C_1$-$C_4$)-alkoxycarbonyl-amino, phenyl which is optionally substituted by up to three identical or different radicals from the series chloro, fluoro, methyl and ($C_1$-$C_4$)-alkoxy, naphthyl, 4,5,6,7-tetrahydrobenzimidazol-2-yl or indolyl, or a physiologically acceptable salt thereof, with the proviso that

a) if E represents a residue of the formula II, $\beta$-Asn, $\beta$-Asp-OMe or D-Thi, F is exclusively Ser($R^1$) and

b) if F represents Leu, Phe or Trp, E is exclusively D-Ser($R^1$), which comprises condensing a fragment with an N-terminal free amino group with a fragment with a C-terminal free carboxyl group, removing one or more protective groups optionally introduced temporarily to protect functional groups and optionally converting the peptide thus obtained into its physiologically acceptable salt.

**2.** The process as claimed in claim 1, wherein a peptide of the formula I is prepared, in which

X is absent;

A is Pgl;

B is His;

C is Trp;
D is Tyr or His;
E is D-Ser($R^1$), $\beta$-Asn, $\beta$-Asp-OMe or the residue of a D-amino acid of the formula II;
F is Ser($R^1$), Trp or Leu and
G is Gly-$NH_2$, Aza-Gly-$NH_2$ or NH-($C_1$-$C_4$)-alkyl and
$R^1$ and $R^2$ are as defined in claim 1, or a physiologically acceptable salt thereof.

**3.** The process as claimed in claim 1, wherein a peptide of the formula I is prepared, in which
X is hydrogen or ($C_1$-$C_7$)-acyl or is absent,
A is dehydro-Pro, Pro, D-Thi, D-Pgl, optionally substituted D-Nal(2), optionally substituted D-Phe or optionally substituted D-Trp;
B is optionally substituted D-Phe;
C is optionally substituted D-Trp, D-Thi or D-Pal(3);
D is Tyr, Arg or His;
E is D-Ser($R^1$), D-Thi or the radical of a D-amino acid of the formula II;
F is Ser($R^1$), Leu, Phe or Trp and
G is Gly-$NH_2$, D-Ala-$NH_2$, Aza-Gly-$NH_2$ or NH-($C_1$-$C_4$)-alkyl and
$R^1$ and $R^2$ are as defined in claim 1;
or a physiologically acceptable salt thereof.

**4.** The process as claimed in claim 3, wherein a peptide of the formula I is prepared, in which
X is ($C_1$-$C_7$)-acyl,
A is D-Nal(2);
B is D-Phe(Cl);
C is D-Trp;
D is Tyr, His or Arg;
E is D-Ser($R^1$);
F is Ser($R^1$), Leu, Phe or Trp and
G is D-Ala-$NH_2$ or Aza-Gly-$NH_2$ and
$R^1$ and $R^2$ are as defined in claim 1,
or a physiologically acceptable salt thereof.

**5.** The process as claimed in any of claims 1-4, wherein a peptide of the formula I is prepared, in which $R^1$ represents an unprotected glycosyl radical, or a physiologically acceptable salt thereof.

**6.** A process for the preparation of a pharmaceutical composition containing a peptide of the formula I as claimed in any of claims 1-4, which comprises bringing the peptide into a suitable form for administration together with a physiologically acceptable carrier and optionally further additives, auxiliaries and/or preservatives.

## Revendications

## Revendications pour les Etats contractants suivants : AT, BE, CH, DE, FR, GB, GR, IT, LI, LU, NL, SE

**1.** Peptide de formule générale I

$$\overset{1}{X}-\overset{2}{A}-\overset{3}{B}-\overset{4}{C}-\overset{5}{Ser}-\overset{6}{D}-\overset{7}{E}-\overset{8}{F}-\overset{9}{Arg}-\overset{10}{Pro}-G \qquad (I)$$

dans laquelle
X représente un atome d'hydrogène ou un groupe acyle en $C_1$-$C_7$, ou, lorsque A représente le groupe pyroglutamyle, est absent;
A représente un reste Pgl, déhydro-Pro, Pro, D-Thi, D-Pgl ou un reste D-Nal(2) éventuellement substitué sur le noyau aromatique par un ou deux substituants identiques ou différents choisis parmi des atomes de brome, chlore, fluor et les groupes nitro, amino, méthyle et méthoxy, un reste D-Phe substitué de cette façon ou D-Trp substitué de cette façon;
B représente un reste His ou un reste D-Phe éventuellement substitué sur le noyau phényle par

un ou deux substituants identiques ou différents, choisis parmi des atomes de brome, chlore, fluor et les groupes nitro, amino, méthyle et méthoxy;

C représente un reste Trp, D-Thi, D-Pal(3) ou un reste D-Trp éventuellement substitué en position 5 et/ou 6 par un ou deux substituants identiques ou différents, choisis parmi des atomes de brome, chlore, fluor et des groupes nitro, amino, méthyle ou méthoxy;

D représente Tyr, Arg ou His;

E représente D-Ser($R^1$), $\beta$-Asn, $\beta$-Asp-OMe, D-Thi ou le reste d'un D-aminoacide de formule générale II

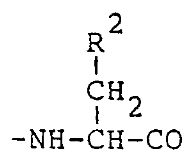

(II)

F représente Ser($R^1$), Leu, Trp ou Phe;

G représente Gly-$NH_2$, Aza-Gly-$NH_2$, D-Ala-$NH_2$ ou un groupe NH-alkyle($C_1$-$C_4$);

$R^1$ représente un reste glycosyle éventuellement partiellement protégé, comportant au moins un groupe hydroxy libre; et

$R^2$ représente un atome d'hydrogène ou un radical alcoxy-($C_1$-$C_4$)-carbonyle, alcoxy en $C_1$-$C_4$, alkyle en $C_1$-$C_4$ qui est éventuellement monosubstitué par un groupe alcoxy-($C_1$-$C_4$)-carbonyle ou alcoxy($C_1$-$C_4$)-carbonylamino, un radical phényle qui est éventuellement substitué par jusqu'à trois substituants identiques ou différents, choisis parmi des atomes de chlore ou de fluor et des groupes méthyle et alcoxy en $C_1$-$C_4$, le radical naphtyle, 4,5,6,7-tétrahydrobenzimidazol-2-yle ou indolyle,

ou sel physiologiquement acceptable de celui-ci, avec la condition que

a) lorsque E représente un reste de formule II, $\beta$-Asn, $\beta$-Asp-OMe ou D-Thi, F a exclusivement la signification de Ser($R^1$) et

b) lorsque F représente Leu, Phe ou Trp, E a exclusivement la signification de D-Ser($R^1$).

**2.** Peptide de formule générale I selon la revendication 1, dans lequel

X est absent;

A représente Pgl;

B représente His;

C représente Trp;

D représente Tyr ou His;

E représente D-Ser($R^1$), $\beta$-Asn, $\beta$-Asp-OMe ou le reste d'un D-aminoacide de formule II;

F représente Ser($R^1$), Trp ou Leu et

G représente Gly-$NH_2$, Aza-Gly-$NH_2$ ou un groupe NH-alkyle($C_1$-$C_4$); et

$R^1$ et $R^2$ sont tels que définis dans la revendication 1;

ou sel physiologiquement acceptable de celui-ci.

**3.** Peptide de formule générale I selon la revendication 1, dans lequel

X représente un atome d'hydrogène ou un groupe acyle en $C_1$-$C_7$, ou est absent;

A représente un reste déhydro-Pro, Pro, D-Thi, D-Pgl, un reste D-Nal(2) éventuellement substitué, D-Phe éventuellement substitué ou D-Trp éventuellement substitué;

B représente un reste D-Phe éventuellement substitué;

C représente un reste D-Trp, D-Thi ou D-Pal(3) éventuellement substitués;

D représente Tyr, Arg ou His;

E représente D-Ser($R^1$), D-Thi ou le reste d'un D-aminoacide de formule II;

F représente Ser($R^1$), Leu, Phe ou Trp et

G représente Gly-$NH_2$, D-Ala-$NH_2$, Aza-Gly-$NH_2$ ou un reste NH-alkyle($C_1$-$C_4$); et

$R^1$ et $R^2$ sont tels que définis dans la revendication 1;

ou sel physiologiquement acceptable de celui-ci.

**4.** Peptide de formule générale I selon la revendication 3, dans lequel

X représente un groupe acyle en $C_1$-$C_7$;

A représente D-Nal(2);

B représente D-Phe(Cl);
C représente D-Trp;
D représente Tyr, His ou Arg;
E représente D-Ser($R^1$);
F représente Ser($R^1$), Leu, Phe ou Trp et
G représente D-Ala-$NH_2$ ou Aza-Gly-$NH_2$; et
$R^1$ et $R^2$ sont tels que définis dans la revendication 1, ou sel physiologiquement acceptable de celui-ci.

5. Peptide de formule générale I selon l'une des revendications 1 à 4, dans lequel $R^1$ représente un radical glycosyle non protégé; ou sel physiologiquement acceptable de celui-ci.

6. Procédé pour la préparation d'un peptide selon l'une des revendications 1 à 5, caractérisé en ce que l'on condense un fragment, à groupe amino libre à l'extrémité N-terminale, avec un fragment à groupe carboxy libre à l'extrémité C-terminale, on élimine un ou plusieurs groupes protecteurs éventuellement introduits temporairement pour la protection de groupes fonctionnels, et éventuellement on convertit le peptide ainsi obtenu en un de ses sels physiologiquement acceptable.

7. Peptide selon l'une des revendications 1 à 4, pour utilisation en tant que médicament.

8. Peptide selon l'une des revendications 1 à 4, pour utilisation en tant qu'agent accroissant la fertilité.

9. Utilisation d'un peptide selon l'une des revendications 1 à 4, en tant qu'agent abaissant le taux plasmatique de gonadotropine, testostérone et oestrogène.

10. Composition pharmaceutique contenant un peptide selon l'une des revendications 1 à 4, ou un sel physiologiquement acceptable de celui-ci.

11. Procédé pour la fabrication d'une composition selon la revendication 10, caractérisé en ce que l'on met sous une forme pharmaceutique appropriée un peptide de formule générale I ou un sel physiologiquement acceptable de celui-ci, conjointement avec un véhicule physiologiquement acceptable et éventuellement d'autres additifs, adjuvants et/ou conservateurs.

**Revendications pour l'Etat contractant suivant : ES**

1. Procédé pour la préparation d'un peptide de formule générale I

```
1 2 3  4   5 6 7  8    9  10
X-A-B-C-Ser-D-E-F-Arg-Pro-G                (I)
```

dans laquelle
X représente un atome d'hydrogène ou un groupe acyle en $C_1$-$C_7$, ou, lorsque A représente le groupe pyroglutamyle, est absent;
A représente un reste Pgl, déhydro-Pro, Pro, D-Thi, D-Pgl ou un reste D-Nal(2) éventuellement substitué sur le noyau aromatique par un ou deux substituants identiques ou différents choisis parmi des atomes de brome, chlore, fluor et les groupes nitro, amino, méthyle et méthoxy, un reste D-Phe substitué de cette façon ou D-Trp substitué de cette façon;
B représente un reste His ou un reste D-Phe éventuellement substitué sur le noyau phényle par un ou deux substituants identiques ou différents, choisis parmi des atomes de brome, chlore, fluor et les groupes nitro, amino, méthyle et méthoxy;
C représente un reste Trp, D-Thi, D-Pal(3) ou un reste D-Trp éventuellement substitué en position 5 et/ou 6 par un ou deux substituants identiques ou différents, choisis parmi des atomes de brome, chlore, fluor et des groupes nitro, amino, méthyle ou méthoxy;
D représente Tyr, Arg ou His;
E représente D-Ser($R^1$), $\beta$-Asn, $\beta$-Asp-OMe, D-Thi ou le reste d'un D-aminoacide de formule générale II

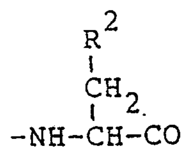

(II)

F représente Ser($R^1$), Leu, Trp ou Phe;

G représente Gly-$NH_2$, Aza-Gly-$NH_2$, D-Ala-$NH_2$ ou un groupe NH-alkyle($C_1$-$C_4$);

$R^1$ représente un reste glycosyle éventuellement partiellement protégé, comportant au moins un groupe hydroxy libre; et

$R^2$ représente un atome d'hydrogène ou un radical alcoxy-($C_1$-$C_4$)-carbonyle, alcoxy en $C_1$-$C_4$, alkyle en $C_1$-$C_4$ qui est éventuellement monosubstitué par un groupe alcoxy-($C_1$-$C_4$)-carbonyle ou alcoxy($C_1$-$C_4$)-carbonylamino, un radical phényle qui est éventuellement substitué par jusqu'à trois substituants identiques ou différents, choisis parmi des atomes de chlore ou de fluor et des groupes méthyle et alcoxy en $C_1$-$C_4$, le radical naphtyle, 4,5,6,7-tétrahydrobenzimidazol-2-yle ou indolyle,

ou d'un sel physiologiquement acceptable de celui-ci, avec la condition que

a) lorsque E représente un reste de formule II, $\beta$-Asn, $\beta$-Asp-OMe ou D-Thi, F a exclusivement la signification de Ser($R^1$) et

b) lorsque F représente Leu, Phe ou Trp, E a exclusivement la signification de D-Ser($R^1$),

caractérisé en ce que l'on condense un fragment, à groupe amino libre à l'extrémité N-terminale, avec un fragment à groupe carboxy libre à l'extrémité C-terminale, on élimine un ou plusieurs groupes protecteurs éventuellement introduits temporairement pour la protection de groupes fonctionnels, et éventuellement on convertit le peptide ainsi obtenu en un de ses sels physiologiquement acceptable.

**2.** Procédé selon la revendication 1, caractérisé en ce que l'on prépare un peptide de formule générale I, dans lequel

X est absent;

A représente Pgl;

B représente His;

C représente Trp;

D représente Tyr ou His;

E représente D-Ser($R^1$), $\beta$-Asn, $\beta$-Asp-OMe ou le reste d'un D-aminoacide de formule II;

F représente Ser($R^1$), Trp ou Leu et

G représente Gly-$NH_2$, Aza-Gly-$NH_2$ ou un groupe NH-alkyle($C_1$-$C_4$); et

$R^1$ et $R^2$ sont tels que définis dans la revendication 1;

ou un sel physiologiquement acceptable de celui-ci.

**3.** Procédé selon la revendication 1, caractérisé en ce que l'on prépare un peptide de formule générale I, dans lequel

X représente un atome d'hydrogène ou un groupe acyle en $C_1$-$C_7$, ou est absent;

A représente un reste déhydro-Pro, Pro, D-Thi, D-Pgl, un reste D-Nal(2) éventuellement substitué, D-Phe éventuellement substitué ou D-Trp éventuellement substitué;

B représente un reste D-Phe éventuellement substitué;

C représente un reste D-Trp, D-Thi ou D-Pal(3) éventuellement substitués;

D représente Tyr, Arg ou His;

E représente D-Ser($R^1$), D-Thi ou le reste d'un D-aminoacide de formule II;

F représente Ser($R^1$), Leu, Phe ou Trp et

G représente Gly-$NH_2$, D-Ala-$NH_2$, Aza-Gly-$NH_2$ ou un reste NH-alkyle($C_1$-$C_4$); et

$R^1$ et $R^2$ sont tels que définis dans la revendication 1;

ou un sel physiologiquement acceptable de celui-ci.

**4.** Procédé selon la revendication 3, caractérisé en ce que l'on prépare un peptide de formule générale I, dans lequel

X représente un groupe acyle en $C_1$-$C_7$;

A représente D-Nal(2);

B représente D-Phe(Cl);

C représente D-Trp;

D représente Tyr, His ou Arg;
E représente D-Ser($R^1$);
F représente Ser($R^1$), Leu, Phe ou Trp et
G représente D-Ala-$NH_2$ ou Aza-Gly-$NH_2$; et
$R^1$ et $R^2$ sont tels que définis dans la revendication 1, ou un sel physiologiquement acceptable de celui-ci.

**5.** Procédé selon l'une des revendications 1 à 4, caractérisé en ce que l'on prépare un peptide de formule générale I, dans lequel $R^1$ représente un radical glycosyle non protégé, ou un sel physiologiquement acceptable de celui-ci.

**6.** Procédé pour la fabrication d'un produit pharmaceutique contenant un peptide de formule générale I selon l'une des revendications 1 à 4, caractérisé en ce que l'on met celui-ci sous une forme pharmaceutique appropriée, conjointement avec un véhicule physiologiquement acceptable et éventuellement d'autres additifs, adjuvants et/ou conservateurs.